# EUROPEAN PATENT APPLICATION

(11) **EP 1 063 296 A1**
(43) Date of publication of application: **27.12.2000**
(21) Application number: 99939199.8
(22) Date of filing: 11.03.1999
(51) Int. Cl.: C12N 15/55, C12N 9/22, C12Q 1/34, A61K 48/00, A61K 38/43

(54) **NUCLEIC ACID ENZYME SHOWING ALLOSTERIC RNA-CLEAVING ACTIVITY ON TARGET RNA**

(30) Priority: 12.03.1998 JP 6096998; 30.10.1998 JP 31109898
(71) Applicant: TAISHO PHARMACEUTICAL CO., LTD, Tokyo 170-8633 (JP); Japan ,represented by Secretary of Agency of Industrial Science and Technology, Chiyoda-ku, Tokyo 100-8921 (JP)
(72) Inventor: TAIRA, Kazunari, Ibaraki 305-0046 (JP); KUWABARA, Tomoko, Ibaraki 305-0044 (JP); HITOSHIO, Akio, Taisho Pharmaceutical Co., Ltd., Tokyo 170-8633 (JP)
(74) Representative: HOFFMANN - EITLE
(86) International application number: JP9901187
(87) International publication number: WO9946388

(57) **Abstract**

A nucleic acid enzyme with allosteric RNA-cleaving activity on a target RNA. An expression vector containing a DNA coding for the nucleic acid enzyme. A method for producing the nucleic acid enzyme, in which an expression vector DNA containing a DNA encoding the nucleic acid enzyme is used as a template in the transcription to RNA. A pharmaceutical composition containing the nucleic acid enzyme or the expression vector containing the DNA coding for the nucleic acid enzyme as an effective component. A method for specifically cleaving target RNA by using the nucleic acid enzyme.

## Description

### FIELD OF THE INVENTION

The present invention relates to a nucleic acid enzyme and a use thereof. More particularly, the present invention relates to a nucleic acid enzyme having allosteric RNA-cleaving activity on target RNA.

### BACKGROUND OF THE INVENTION

In early 1980's, the self-splicing of rRNA of Tetrahymena pyriformis found by Cech et al., Colorado University, United States (K. Kruger, P.J. Grabowski, A.J. Zaug, J. Sands, D.E. Gottschling, T.R. Cech, Cell, 31, 147-157 (1982)) and the analysis of ribonuclease P, a complex enzyme composed of RNA and protein, by Altman, Yale University, United States (C.Guerrier-Takada, K. Gaydiner, T. Marsh, N. Pace, S. Altman, Cell, 35, 849-857 (1983)) led to a finding of a ribozyme (ribonucleotide acid + enzyme) which is an RNA with a catalytic function. Since then, various ribozymes have been found (R.H. Symons, Trend. Biochem. Sci., 14, 445-450 (1989); R.H. Symons, Annu. Rev. Biochem., 61, 641-671 (1992); J. Bratty, P. Chartrand, G. Ferbeyre, R. Cedergren, Biochim. Biophys. Acta, 1216, 345-359 (1993); Hasehoff. J and W.L. Gerlach, Nature, 334, 585-591 (1988); C.J. Hutchins, P.D. Rathjen, A.C. Forster, R.H. Symons, Nucleic Acids. Res., 14, 3627-3640 (1986)), which, together with reverse transcriptase, the finding of intron, RNA editing and the like, posed a question to the then established idea of central dogma. At the same time, an RNA that had been understood to do nothing but to intermediate information between DNA and protein was found to have both (genetic) information and (catalytic) function. Thus, RNA molecules were focused as a central molecule for the "RNA world" theory which states that the RNA molecule is indeed the origin of life (G.F. Joyce, Nature, 338 217-224 (1989); N.R. Pace, T.L. Marsh, Origins of Life, 16, 97 (1985); A. Lazcano, R. Guerrero, J. Oro, J. Mol. Evol., 27, 283 (1988); L.E. Orgel, Nature, 358, 203 (1992); R.F. Gesteland, J.F. Atkins, The RNA World, Monograph 24, Cold Spring Harbor Laboratory Press, Plain view, New York (1993)).

Among all, hammerhead ribozymes are one of the most well researched ribozymes. The hammerhead ribozyme which functions to give self-splicing reaction (cis-type) in nature (T.R. Cech, Annu. Rev. Biochem., 59, 543 (1990); A.C. Foster, R.H. Symons, Cell, 49, 211 (1987); A.C. Jefferies, R.H. Symons, Nucleic Acids Res., 17, 1371 (1989)) was divided into two RNA strands (a substrate region and an enzymatic activity retaining region) (i.e., converted to trans-type) by the groups of Uhlenbeck et al., Haseloff and Gerlach et al. (O.C. Uhlenbeck, Nature, 328, 596 (1987); J. Hasehoff, W.L. Gerlach, Nature, 334, 585 (1988)), whereby ribozyme was suggested as a candidate in an application to genetic therapy. Since then, numerous applied researches targeting cancers and AIDS have been reported (M. Cotten, M.L. Bimatiel, EMBO J, 8, 861 (1989); N. Sarver, E. Cantin, O. Chang, O. Lande, D. Stephens, J. Zaia, J. Rossi, Science 247, 1222 (1990); M. Homann, M. Tzortzakari, K. Rittner, S. Sczakiel, M. Tabler, Nucleic Acids Res 21, 2809 (1993); R.C. Mulligan, Science O, 926 (1993); S. Altman: Proc. Natl. Acad. Sci. USA, 90. 10898 (1993); P. Marschall, J.B. Thompson, F. Eckstein, Cell. Mol. Neurobiol., 14, 523 (1994); S.M. Sullivan, J. Invest. Dermatol., 103, 85 (1994); F.H. Cameron, P.A. Jennings, Antisense Res. Dev., 4, 87 (1994); L.Q. Sun, D. Warrilow, L. Wang, C. Witherington, J. Macpherson, G. Symonds, Proc. Natl. Acad. Sci. USA, 91, 9715 (1994); R.E. Christoffersen, J.J. Marr, J. Med. Chem. 38, 2023 (1995); G. Ferbeyre, J. Bratty, H. Chen, R. Cedergern, Gene 155, 45 (1995); M. Kiehntopf, E.L. Eaquivel, M.A. Brach, F. Herrmann, J. Mol. Med., 73, 65 (1995); J.D. Thompson, D. Macejak, L. Couture, D.T. Stinchcomb, Nat. Med. 1, 277 (1995); T. Tuschl, J.B. Thomson, F. Eckstein, Curr. Opin. Struct. Biol. 5, 296 (1995)).

A ribozyme binds to a substrate RNA by forming complementary base pairs with the substrate. Then, the substrate RNA molecule is cleaved in the presence of magnesium indispensable for the reaction. Since both substrate-binding regions (stems I and III) recognize the substrate by appropriately forming base pairs with the corresponding substrate sequences, the ribozyme has an extremely high substrate-specificity. The extremely high substrate-specificity causes least side effects in cells, if any, and thus is very advantageous for using the ribozyme as a gene expression inhibitor.

There is, however, an exception to the high substrate-specificity of ribozymes, when the target substrate is a chimera (two or more distinct gene sequences binding to function as a unitary gene). When a mis-splicing occurs to give another chimeric sequence (exon 1-exon 3) where a sequence (exon 2) should join with another sequence (exon 3) and causes a disease such as a cancer, ribozymes may naturally be thought to be used for a gene therapy to suppress this abnormal expression of RNA. Since the sequences themselves (exons 1, 2 and 3) are normal messages, it is important to specifically cleave only the mRNA with the abnormal junction sequence (exon 1-exon 3) to suppress the expression thereof. Accordingly, the ribozyme used should not give any influence to the normal mRNA (exon 2-exon 3).

When a conventional hammerhead ribozyme is used for suppressing such a gene expression, no problem occurs if a GUC triplet (generally NUX (N; A, G, C, U X; A, C, U)), a sequence cleavable with the ribozyme, exists at the junction site of exon 1-exon 3 sequences specific to the abnormal mRNA. However, such a sequence cleavable with the ribozyme rarely exists at or near the junction site, unfortunately. When there is no cleavable site at the junction site, a cleavable sequence remote from the junction site is inevitably targeted. Since the cleavable sites remote from the junction sites exist in both normal and abnormal mRNAs, non-specific cleavage in normal mRNA is inevitable. Even when an NUX sequence cleavable with a ribozyme is present at or near the junction site, there is even smaller possibility that it is a triplet (GUC triplet) that can preferentially and efficiently be cleaved with a hammerhead ribozyme. Therefore, it has been difficult to create a ribozyme with high specificity even in the above-described case, while retaining a high cleaving activity.

Notable examples of cases where certain chimeric mRNA causes actual diseases are the formations of Philadelphia chromosome which causes CML (chronic myelocytic leukemia) and ALL (acute lymphocytic leukemia). In these leukemia, a reciprocal chromosomal translocation t(9;22) (q34;q11) takes place to produce a *BCR-ABL* fusion gene. In the case of CML, two types of reciprocal chromosomal translocations, K28 and L6 translocations, result in two types of *BCR-ABL* fusion genes, and finally produce two types of chimeric mRNAs due to splicing (K28-junction (b3a2) mRNA and L6-junction (b2a2) mRNA). One of them has a sequence (GUU triplet) near the *BCR-ABL* junction site, which can be cleaved with a hammerhead ribozyme (K28-junction (b3a2) mRNA), while the other mRNA does not include a potential cleavable sequence near the junction site (L6-junction (b2a2) mRNA). Accordingly, expression of the latter mRNA cannot specifically be inhibited with conventional hammerhead ribozymes. Most of the attempts reported so far for inhibiting expression of abnormal protein (p210^{*BCR-ABL*}) from L6 mRNA add a long antisense moiety to the hammerhead ribozyme, and bind the resultant to the complementary junction site to attain cleavage specificity. However, when the substrate-binding moiety of the ribozyme becomes longer by the addition of the long antisense moiety, the dissociation of the ribozyme from the substrate following the binding of the substrate becomes very slow. As a result, the cleavage efficiency becomes lower because of the poor turnover of the enzyme. Even worse, the substrate-specificity that can be obtained with such efforts is not so high as expected. This is because the too long antisense moiety partially binds to normal mRNAs such as *ABL* mRNA and *BCR* mRNA and weakens the substrate recognizing ability of the ribozyme. Thus, non-specific cleavage of normal mRNAs has been unavoidable.

Accordingly, the present invention has an objective of providing a nucleic acid enzyme having allosteric cleaving activity on a substrate.

The present invention also has an objective of providing an expression vector containing DNA coding for the above-mentioned nucleic acid enzyme.

The present invention still has an objective of providing a pharmaceutical composition comprising the above-mentioned nucleic acid enzyme or DNA coding for the nucleic acid enzyme, as an effective component.

The present invention yet still has an objective of providing a method for producing and using the above-described nucleic acid enzyme.

### DISCLOSURE OF THE INVENTION

The present inventors has completed the present invention by constructing a nucleic acid enzyme (ribozyme) which has one active center region and two substrate-binding regions, where one substrate-binding region binds to a junction site of L6 (b2a2) chimeric mRNA while the other region binds to an convenient cleavage sequence remote from the junction site to cleave the substrate downsteam of the cleavage sequence. In other words, the present invention is a nucleic acid enzyme with an allosteric RNA-cleaving activity on target RNAS. The nucleic acid enzyme of the invention may preferably comprise a dimeric structure formed by an RNA molecule containing the following nucleotide sequence (10) and an RNA molecule containing the following nucleotide sequence (20),
5'X¹ₗ...X¹ₕ Y¹ₗ...Y¹ᵢ Z¹ₗ...Z¹ⱼ 3' (10)
5'Z²ₗ...Z²ₙ Y²ₗ...Y²ₘ X²ₗ...X²ₖ 3' (20)

(wherein X¹ₗ-X¹ₕ, X²ₗ-X²ₖ, Y¹ₗ-Y¹ᵢ, Y²ₗ-Y²ₘ, Z¹ₗ-Z¹ⱼ and Z²ₗ-Z²ₙ are independently any one of A, U, T, C and G;
h and k are integers of 1 or higher (e.g., an integer of 1-100);
i and m are integers of 1 or higher (e.g., an integer of 1-100);
j is an integer of 1 or higher (e.g., an integer of 1-100);
n is an integer of 1 or higher (e.g., an integer of 1-100);
X¹ₗ...X¹ₕ and X²ₗ...X²ₖ are nucleotide sequences complementary to a specific sequence in the target RNA;
Y¹ₗ...Y¹ᵢ and Y²ₗ...Y²ₘ are nucleotide sequences forming stems; and
Z¹ₗ...Z¹ⱼ and Z²ₗ...Z²ₙ are nucleotide sequences containing a region complementary to a sequence near a cleavage site of the target RNA and a region capable of forming a cavity for capturing Mg²⁺ ion only in the presence of the target RNA).
The target RNAs include chimeric mRNAs causative of a disease. The chimeric mRNA may be L6 (b2a2) chimeric mRNA causative of chronic myelocytic leukemia. The nucleic acid enzyme of the invention may comprise a dimeric structure formed by an RNA molecule containing the following nucleotide sequence (1) and an RNA molecule containing the following nucleotide sequence (2),
5'GAAGGGCUUC UUUCAUCGAA ACCCUGAGG 3' (1) (SEQ ID NO:1)
5'CACUCACUGA UGAGAGUUAU UGAUGGUCAG 3' (2) (SEQ ID NO:2)
(wherein nucleotides 21-29 of the nucleotide sequence (1) and nucleotides 17-31 of the nucleotide sequence (2) may be modified to conform complementation with the sequence near the cleavage site of the target RNA).

A linker sequence and a tRNA^{Val} promoter may be added upstream of each of the nucleotide sequences (1) and (2). The linker sequence added upstream of the nucleotide sequence (1) may contain the following nucleotide sequence (3), and the linker sequence added upstream of the nucleotide sequence (2) may contain the following nucleotide sequence (4),
5'AAA 3' (3)
5'UUU 3' (4).

In addition, the tRNA^{Val} promoter sequence added upstream of each of the nucleotide sequences (1) and (2) may contain the following nucleotide sequence (5),
5'ACCGUUGGUU UCCGUAGUGU AGUGGUUAUC ACGUUCGCCU AACACGCGAA AGGUCCCCGG UUCGAAACCG GGCACUACAA AAACCAAC 3' (5) (SEQ ID NO:3).

Furthermore, an additional sequence and a terminator sequence may be added downstream of each of the nucleotide sequences (1) and (2). The additional sequence added downstream of the nucleotide sequence (1) may contain the following nucleotide sequence (6), the additional sequence added downstream of the nucleotide sequence (2) may contain the following nucleotide sequence (7), and the terminator sequence added downstream of each of the nucleotide sequences (1) and (2) may contain the following nucleotide sequence (8),
5'AAA 3' (6)
5'AACCGUA 3' (7)
5'UUUUU 3' (8).

The target RNA may be an abnormal mRNA causative of a disease. Examples include abnormal mRNAs causative of HIV (AIDS), acute lymphocytic leukemia, acute promyelocytic leukemia and the like.

The present invention also provides an expression vector comprising a DNA coding for the above-mentioned nucleic acid enzyme.

The present invention further provides a method for producing the above-mentioned nucleic acid enzyme, in which an expression vector DNA containing a DNA encoding the nucleic acid enzyme is used as a template in the transcription to RNA.

The present invention further provides a pharmaceutical composition comprising the above-mentioned nucleic acid enzyme or the expression vector containing a DNA coding for the nucleic acid enzyme as an effective component. The pharmaceutical composition may be for preventing and/or treating a disease caused by the target RNA. Examples of the diseases caused by the target RNA include diseases caused by Philadelphia chromosome abnormality, such as chronic myelocytic leukemia. The pharmaceutical composition of the invention may be used to suppress or inhibit expression of chimeric mRNAS or abnormal mRNAs causative of a disease by expressing the above-mentioned nucleic acid enzyme *in vivo*.

The present invention further provides a method for specifically cleaving the target RNA by using the above-mentioned nucleic acid enzyme. The target RNA may be chimeric mRNAs causative of a disease. Such a disease may be caused by Philadelphia chromosome, such as chronic myelocytic leukemia and acute lymphocytic leukemia. Alternatively, the target RNA may be an abnormal mRNA causative of a disease. Such a disease may be HIV (AIDS), acute lymphocytic leukemia and chronic myelocytic leukemia.

Hereinafter, the present invention will be described in more detail.

### 1. Construction of dimeric minizyme

First, a hammerhead ribozyme, a minizyme, and a dimeric minizyme having a very high activity, which is introduced herein, will be described as to their designs and production processes.

### 1-1 Hammerhead ribozyme -metalloenzyme-

Before describing about the minizymes, hammerhead ribozymes will first be described briefly.

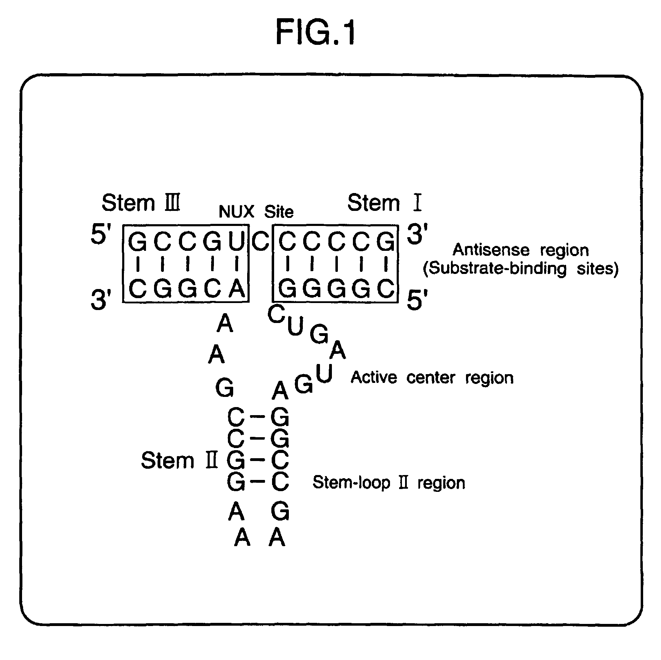

Hammerhead ribozymes were named after their secondary structural shapes of the RNA resembling a hammerhead (Haseoff. J and W.L. Gerlach, Nature, 334, 585-591 (1988); C.J. Hutchins, P.D. Rathjen, A.C. Forster, R.H. Symons, Nucleic Acids. Res., 14, 3627-3640 (1986)). Hammerhead ribozymes have been studied broadly in both basic and applied viewpoints and are typical ribozymes with RNA strand cleaving activity. Self-splicing RNA8 were found during the courses of replications of viroid, which is a virus that infects plants (the smallest pathogen known so far, a single strand circular RNA without a capsid), virusoid (a single strand circular RNA present in RNA viruses, which is not infective alone) and satellite RNA that infects plants with the aid of viroids. When only a segment required for activity was re-constructed *in vitro*, a secondary structure with high homology, i.e., a hammerhead structure, was found (Hasehoff. J and W.L. Gerlach, Nature, 334, 585-591 (1988); C.J. Hutchins, P.D. Rathjen, A.C. Forster, R.H. Symons, Nucleic Acids. Res., 14, 3627-3640 (1986); T.R. Cech, Annu. Rev. Biochem., 59, 543 (1990); A.C. Foster, R.H. Symons, Cell, 49, 211 (1987); A.C. Jeffries, R.H. Symons, Nucleic Acids Res., 17, 1371 (1989); A.C. Foster, R.H. Symons, Cell, 50, 9 (1987)). Thereafter, a nucleotide sequence preserved among various hammerhead ribozymes was found, and was converted to trans-type as described above to give relatively preserved enzymatically-active region and a substrate region that is not preserved (Figure 1) (O.C. Uhlenbeck, Nature, 328, 596 (1987); J. Hasehoff, W.L. Gerlach, Nature, 334, 585 (1988)). Accordingly, a hammerhead ribozyme consists of an antisense region that recognizes and binds the substrate RNA, an active center region forming a loop (cavity) in the vicinity of the antisense region, and a stem-loop II region annexed to the loop. A set law of a three-nucleotide sequence (triplet) was found as a cleavable sequence on the substrate RNA, and it was found that cleavage takes place only after the NUX sequence (M. Koizumi, S. Iwai, E. Ohtsuka, FEBS Lett., 228 (1988); D.E. Ruffer, G.D. Stormo, O.C. Uhlenbeck, Biochemistry, 29, 10695 (1990); C.C. Sheldon, R.H. Symons, Nucleic Acids Res., 17, 5679 (1989); R. Perriman, A. Delver, W.L. Gerlach, Gene, 113, 157 (1992); T. Shimayama, S. Nishikawa, K. Taira, Biochemistry, 34, 3649 (1995); M. Zoumadakis, M. Tabler, Nucleic Acids Res., 23, 1192 (1995)). According to this NUX rule, a GUC sequence triplet has the highest cleavage efficiency, and this GUC is usually mentioned as a typical sequence cleaved by a ribozyme. Other than this triplet, a sequence in the antisense region which can complementarily bind to the substrate may freely be designed according to the nucleotide sequence of the substrate. In other words, the ribozyme may site-specifically cleave any RNA sequence depending on the design thereof. Thus, ribozymes can be applied to gene therapy of a specific gene, as an expression inhibitor of the gene. In a sense, a ribozyme may be referred to as a "molecule scissors" which can freely cleave a "target RNA strand".

What is important here is that a divalent cation such as magnesium ion is essential for the above-described RNA-strand-cleaving activity of the hammerhead ribozyme. Magnesium ion is also necessary for the ribozyme to form an active-type structure, although details thereof as to its involvement in the reaction mechanism is not yet sufficiently elucidated. However, from the experimental results obtained so far, it was found that it is the magnesium ion that is actually performing the cleavage of the RNA strand and that the ribozyme only provides an anchor for the metal ion (S.C. Dahm, W.B. Derrick, O.C. Uhlenbeck, Biochemistry 32, 13040 (1993); J.A. Piccirilli, J.S. Vyle, Nature 361, 85 (1993); Michael, Yarus, FASEB J., 7, 31 (1993); T. Uchimaru, M. Uebayasi, K. Tanabe, K. Taira, FASEB J7, 137 (1993); T.A. Sreitz, J.A. Steitz, Proc. Natl. Acad. Sci. USA, 90, 6498 (1993); M.A. Pyle, Science, 261, 709 (1993); T. Uebayasi, T. Uchimaru, T. Koguma, T. Sawata, S. Shimayama, K. Taira, J. Org. Chem., 59. 7414 (1994); S. Sawata, M. Komiyama, K. Taira, J. Am. Chem. Soc., 117, 2357 (1995); P.K.R. Kumar, D.M. Zhou, K. Taira, Nucleic Acids and Molecular Biology, 10, 217, (1996)). In other words, ribozymes are metalloenzymes. Among the above-mentioned several regions forming the hammerhead ribozyme, the loop segment near the cleavage site is thought to actually provide the anchor. Regions other than this loop for capturing the metal ion may be modified in various ways. For example, the substrate-binding region may be replaced with DNA which is more stable than RNA. The stem-loop II region may possibly be deleted as described below for minimization (J. Goodchild, V. Kohli, Arch. Biochem. Biophys., 284, 386 (1991); M.J. McCall, P. Hendry, P.A. Jennings, Proc. Natl. Acad. Sci. USA, 89, 5710 (1992); J.B. Thompson, T.Tuschl, F. Ekstein, Nucleic Acids Res., 21, 5600 (1993); D. Fu, F. Benseler, L.W. McLaughlin, J. Am. Chem. Soc., 116, 4591 (1994); D.M. Long, O.C. Uhlenbeck, Proc. Natl. Acad. Sci. USA, 91, 6977 (1994)).

### 1-2 Minizyme

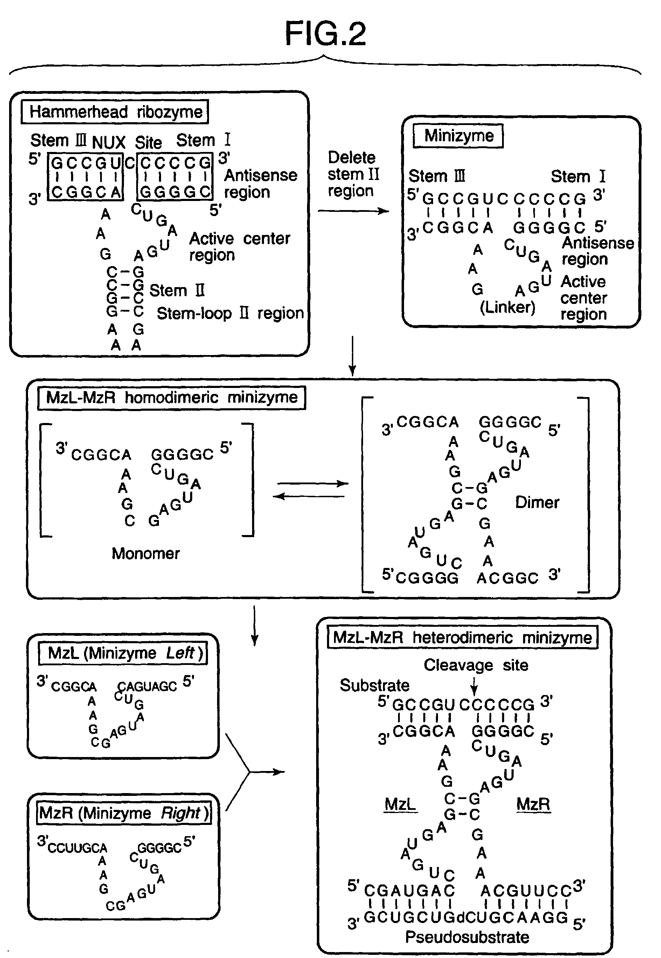

The process of constructing a dimeric minizyme is shown in Figure 2. Processes of constructing a minizyme and a dimeric minizyme will be described with reference to this figure.

As stated above, a minizyme is a minimized hammerhead ribozyme. This minimization is generally carried out by replacing the stem-loop II region of a hammerhead ribozyme (consisting of the antisense regions (stems I and III), the activity center region and the stem-loop II region) with a short chain linker. In an early attempt, the stem-loop II region was deleted. This was carried out to study the role of each region of the hammerhead ribozyme in terms of cleaving activity. As a result, when the stem-loop II region was deleted in its entirety, the cleaving activity was found to be remarkably decreased. Since then, various lengths of nucleotides were studied to substitute for the stem-loop II region in order to construct a minimized ribozyme retaining the cleaving activity. For example, one attempt to construct a minizyme is, instead of deleting the whole stem-loop II region, to substitute a four-residue nucleotide tetraloop therefor. However, the recently reported detailed analysis indicates that the decrease in the number of nucleic acid bases in the stem-loop II region significantly influences the cleaving activity thereof. In fact, the cleaving activity of the various minizymes reported so far is below one-hundredth (and not a few of them are below one-thousandth) of the cleaving activity of a wild-type hammerhead ribozyme (J. Goodchild, V. Kohli, Arch. Biochem. Biophys., 284, 386 (1991); M.J. McCall, P. Hendry, P.A. Jennings, Proc. Natl. Acad. Sci. USA, 89, 5710 (1992); J.B. Thompson, T. Tuschl, F. Ekstein, Nucleic Acids Res., 21, 5600 (1993); D. Fu, F. Benseler, L.W. McLaughlin, J. Am. Chem. Soc., 116, 4591 (1994); D.M. Long, O.C. Uhlenbeck, Proc. Natl. Acad. Sci. USA., 91, 6977 (1994)). Deletion or decrease of the stem-loop II region is considered to disrupt the active-type structure of the ribozyme which is requisite for the ribozyme to carry out the cleavage reaction. Accordingly, minizymes have been considered unpractical to be applied as antiviral drugs or to gene therapy as compared to a conventional hammerhead ribozyme, and thus have been out of interest to many researchers in the art.

### 1-3 Construction of dimeric minizyme -Highly active minizyme-

When the present inventors also have constructed minizymes as described in section 1-2 above, a ribozyme with a very high activity (60% or higher of that of the wild-type ribozyme (k_{cat} = 2.5 min⁻¹: meaning that 1-molecular ribozyme cleaves 2.5 molecules of the substrate per minute)) was fortunately obtained (S.V. Amontov, K. Taira, J. Am. Chem. Soc., 118, 1624 (1996)). Experiments under varied concentrations suggested a reason for this high activity of the minizyme. Specifically, the minizyme was thought in nucleotide sequence level to function under completely different mechanisms, as a dimer (a homodimer; a dimer consisting of two binding molecules having identical sequences) at a higher concentration region. In order to study whether or not the minizyme functions as a dimer, a heterodimeric minizyme which does not exhibit activity unless it forms a dimeric structure was designed (Figure 2, bottom panel). This heterodimeric minizyme is formed by binding MzL (minizyme *Left*) and MzR (minizyme *Right*) which are heterologous molecules with different sequences. The minizyme is designed such that when the minizyme, without forming a dimeric structure, tends to bind to the substrate by itself like a conventional hammerhead ribozyme, only one of the sequences in the substrate-binding region (either stern I or III in the antisense region which complementarily binds to the substrate) forms base pairs with the substrate so that neither an appropriate binding nor cleavage reaction takes place.

In an actual cleavage experiment, cleaving activity was clearly observed. The minizyme having significantly high activity is concluded to function as a dimer (S.V. Amontov, K. Taira, J. Am. Chem. Soc., 118, 1624 (1996)). This newly constructed dimeric minizyme, like hammerhead ribozymes, requires magnesium ion, and relies on GC base pairs in the stem II moiety forming the dimer (S.V. Amontov, K. Taira. J. Am. Chem. Soc., 118, 1624 (1996); S.V. Amontov, S. Nishikawa, K. Taira, FEBS Lett., 386, 99 (1996)).

### 2. Construction of a dimeric minizyme that simultaneously cleaves at two sites of a substrate RNA molecule

Hereinafter, a process for constructing a completely novel type of ribozyme, "a dimeric minizyme which simultaneously cleaves at two sites of a substrate RNA molecule" will be described (T. Kuwabara, S.V. Amontov, M. Warashina, J. Ohkawa, K. Taira, Nucleic Acids Res., 24, 2302 (1996)).

### 2-1 Merit of the ribozyme that simultaneously cleaves at two sites of a substrate RNA molecule

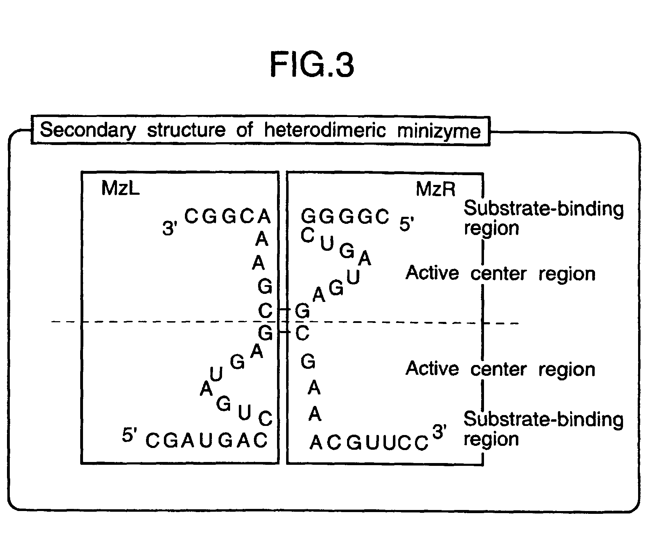

As shown in Figure 3, the dimeric minizyme constructed as described above forms a dimer by binding MzL and MzR which are heterologous molecules with different sequences. When the upper and lower parts (as divided by a dotted line) of this dimer are separately observed, one can realize that each part independently has an active center region as an anchor for capturing magnesium ion necessary for the cleaving activity of a ribozyme (a metalloenzyme) and a substrate-binding region for determining which site of the substrate RNA sequence is to be cleaved. Based on this observation, this dimeric minizyme may simultaneously bind to two sites of a substrate and cleave after the two NUX triplets.

One merit of this construction is of course the increase in the efficiency of cleaving the substrate as compared to that of a conventional ribozyme with only one cleavage site. Other additional and remarkable merit is that, if one of the substrate-binding regions of the dimeric minizyme can have a sequence that can easily bind to a substrate (i.e., if the kₘ value of binding between the substrate and the ribozyme is sufficiently low), the minizyme possibly efficiently cleave at two sites even when the other substrate-binding region give high Kₘ value at a site other than GUC (i.e., an unstable sequence which does not easily bind to the substrate) as long as a triplet with high k_{cat} value is selected. Once one of the substrate-binding regions is bound with the substrate, the binding between the other substrate-binding region and the substrate becomes the same as an intramolecular. Accordingly, the collision rate becomes significantly higher than that of intermolecular reactions.

### 2-2 Dimeric minizyme which specifically cleaves BCR-ABL chimeric mRNA

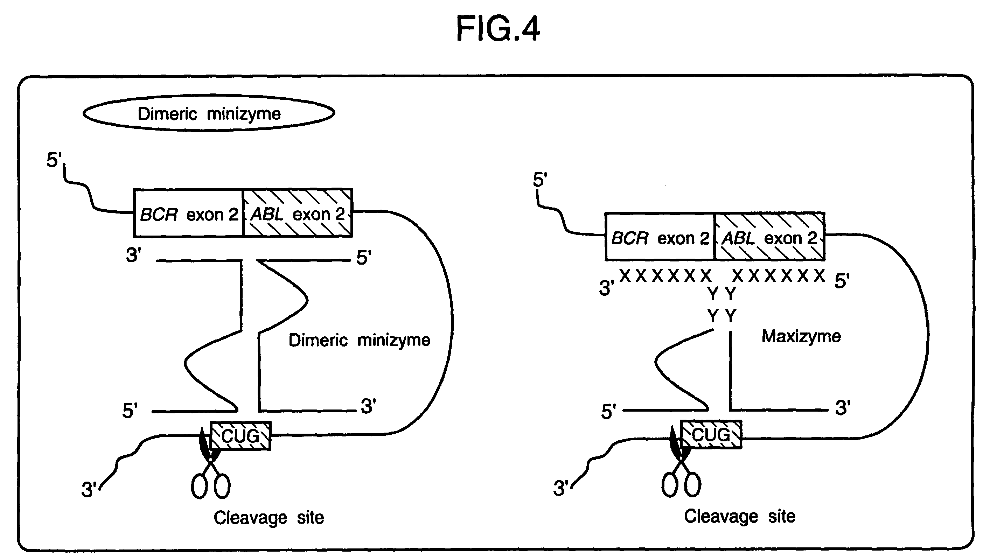

A dimeric minizyme with a dimeric structure has two active center regions and two substrate-binding regions. Thus, it may be possible to construct a system which binds to a junction site of abnormal *BCR-ABL* chimeric mRNA at one substrate-binding region while the other substrate-binding region binds to the most efficient cleavage sequence remote from the junction to cleave the substrate after the (GUC) triplet (Figure 4). Specifically, one of the substrate-binding regions acts as an "eye" for recognizing an abnormal substrate while the other substrate-binding region acts as an arm bearing the "essential" function as a ribozyme to cleave the substrate.

It is important in this construction to form a stable dimer structure capable of expressing activity as a ribozyme, which is valid only when one of the substrate-binding regions of the dimeric minizyme binds to the junction site of the abnormal *BCR-ABL* chimeric mRNA. The stability of the dimer depends on formation of the base pairs in the stem II moiety. When the stability of the base pairs is too high, the dimeric minizyme forms a dimeric structure by itself (without a substrate). Consequently, the substrate-binding region (of the dimeric minizyme) for cleaving the substrate binds to the target substrate region regardless of whether or not the other substrate-binding region as the "eye" for recognizing the substrate functions. Since the sequence to be cleaved also exists in normal mRNAs such as *ABL mRNA* and *BCR mRNA*, non-specific cleavage which has been the problem associated with a conventional hammerhead ribozyme takes place. On the other hand, when the stability of the base pairs in the stem II moiety is too low, a dimeric structure of an active type is hardly formed, resulting in very low substrate-cleaving efficiency which has been another problem associated with a conventional minizyme. The key of constructing the system, in order to overcome both of the problems, relies on a design of the nucleotide sequence to obtain a delicate stability of the base pairs in the stem II moiety and to form an active-type ribozyme only in the presence of abnormal mRNAs.

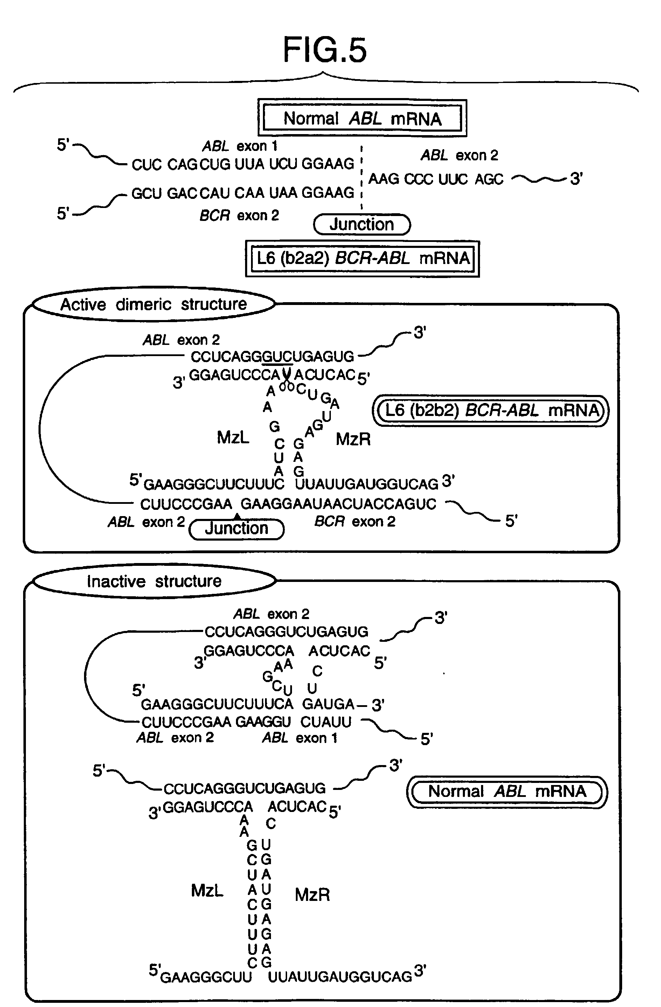

According to this system, the substrate-binding region that recognizes abnormal mRNAs and binds thereto specifically does not cleave the substrate. Therefore, the sequence of the active center region providing an anchor for capturing metal ion indispensable for RNA chain cleavage may be deleted, thereby further minimizing the ordinary dimeric minizyme (Figure 4, right). This further minimized dimeric minizyme was named "maxizyme" in order to distinguish from a conventional monomer minizyme with extremely low activity. "Maxizyme" stands for minimized, active, x-shaped (heterodimeric) and intelligent (allosterically controllable) ribozyme. A maxizyme which was actually designed to specifically cleave *BCR-ABL* chimeric mRNA is shown in Figure 5. As can be appreciated from the figure, the active-type dimeric structure is formed only when the two substrate-binding regions of the dimeric minizyme bind to the target *BCR-ABL* chimeric mRNA (Figure 5, center). The maxizyme is designed such that only an inactive-type structure is formed in the presence of untargeted normal *ABL* mRNAs. When normal *ABL* mRNA sequence comes close to the substrate-binding region that recognizes the junction, the maxizyme forms an inactive-type structure (Figure 5, bottom panel, upper structure). As can be appreciated from the figure, the structure of the active center site for capturing magnesium ion necessary for the cleaving activity of the hammerhead ribozyme is disrupted, unlike that of the active-type dimeric structure. As a result, the dimeric minizyme does not cause non-specific cleavage in normal *ABL* mRNAs. When the substrate-binding region for cleaving the substrate is bound to the substrate alone (in which case, the substrate bound may be either normal *ABL* mRNA or *BCR-ABL* chimeric mRNA), a structure with a closed active site is formed (Figure 5, bottom panel, lower structure), unless the target *BCR-ABL* chimeric mRNA sequence is assigned to the other substrate-binding region. Since magnesium ion indispensable for cleavage cannot be captured, cleavage does not take place.

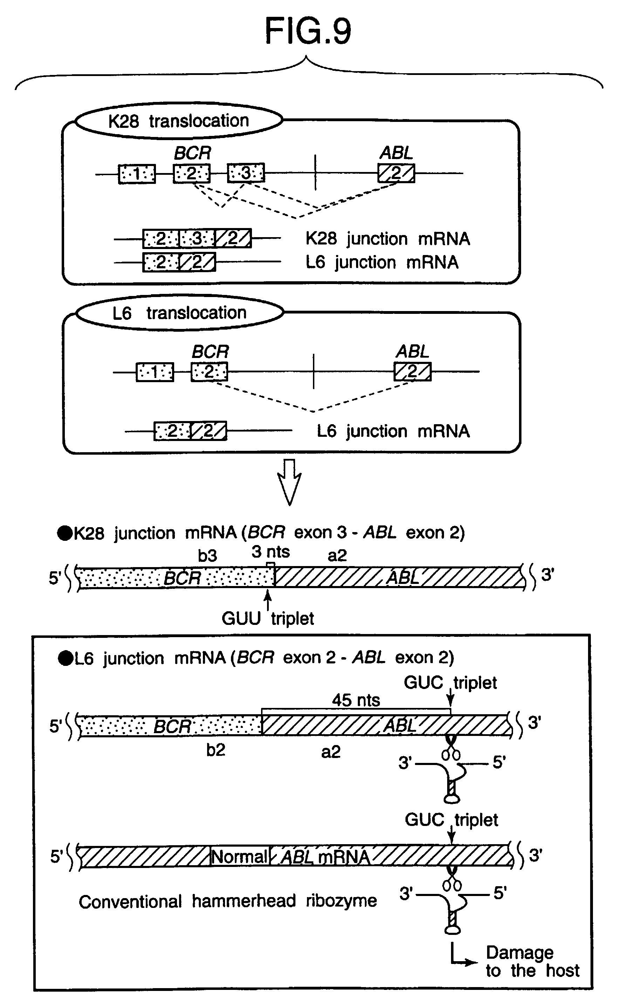

The translated product of *BCR-ABL* fusion mRNAS causes chronic myelocytic leukemia (CML), which is a clonal rnyeloproliferative disorder of hematopoietic stem cells that is associated with the Philadelphia chromosome (Nowell and Hungerford, 1960). The reciprocal chromosomal translocation t(9;22) (q34;q11) can be subdivided into two types, K28 and L6 translocations which produce a *BCR-ABL* fusion gene. These genes code for two types of mRNAs, namely, b3a2 (consisting of *BCR* exon 3 and *ABL* exon 2) and b2a2 (consisting of *BCR* exon 2 and *ABL* exon 2) (Figure 9; Rowley, 1973; Bartram et al., 1983; Heisterkamp et al., 1983; Groffen et al., 1984; Shtivelman et al., 1985, 1986). Both of these mRNAs are translated into a protein of 210 kDa (p210^{*BCR-ABL*}), which is unique to the phenotype of the above-mentioned malignant cell (Konopka et al., 1984).

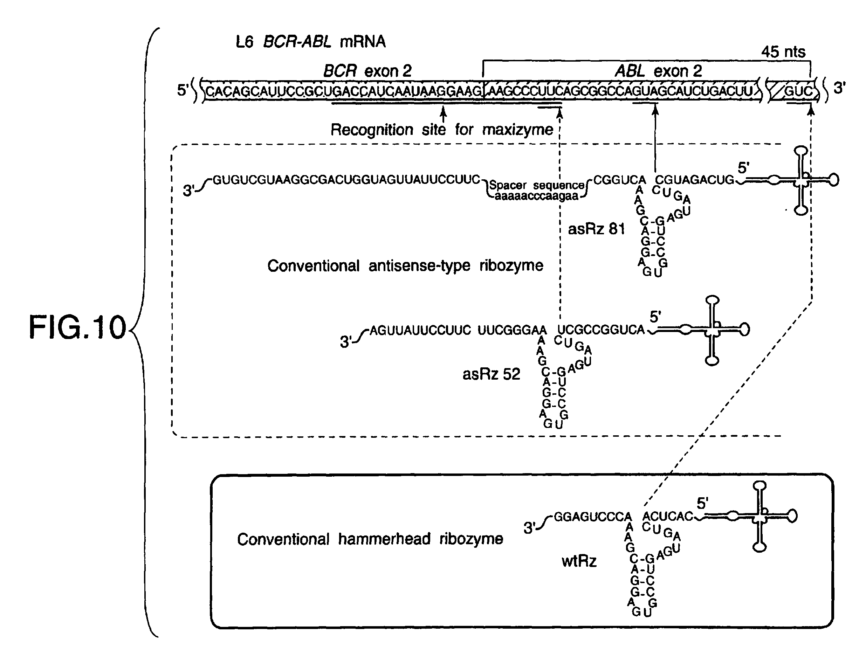

In order to design a ribozyme that can disrupt chimeric RNAs, the junction sequence must be targeted. Otherwise, a normal RNA sharing the part with the chimeric RNAs will also be cleaved by the ribozyme, thereby damaging a host cell (Figure 9, bottom panel). In the case of K28 *BCR-ABL* chimeric RNA sequence b3a2, the GUU triplet which is a potential site of cleavage by the ribozyme is located three nucleotides upstream from the chimeric junction. Thus, a hammerhead ribozyme designed according to a conventional method might be expected to specifically cleave the abnomal mRNA generated from K28 translocations. In fact, several examples of such a cleavage have been reported (Shore et al., 1993; Snyder et al., 1993; Lange et al., 1993, 1994; Wright et al., 1993; Kearney et al., 1995; Leopold et al., 1995; Kronenwett et al., 1996). On the other hand, in the case of b2a2 sequence resulting from L6 translocations and some of K28 translocations, a triplet sequence cleavable with a hammerhead ribozyme is absent within two or three nucleotides from the junction in question. In general, GUC triplet is most susceptible to cleavage by a hammerhead ribozyme while one such triplet is present 45 nucleotides from the junction. When this GUC triplet is cleaved by a ribozyme (wtRz; Figure 10), normal *ABL* mRNA sharing the sequence in part with abnormal *BCR-ABL* mRNA is also cleaved by the ribozyme, thereby damaging a host cell. In designing a ribozyme capable of cleaving b2a2 mRNA, one must be sure to avoid cleavage of normal *ABL* mRNAs.

Previous attempts to cleave L6 *BCR-ABL* (b2a2) mRNA have required a combination of a long antisense arm and a ribozyme sequence (Pachuk et al., 1994; James et al., 1996). Antisense sequences of about 10-30 nucleotides having a potential of binding to and covering the junction region for some distance beyond the cleavage site were connected to one of the substrate-binding sites of the hammerhead ribozyme. The lengths of the annealing arms are important for the activity of the ribozyme because they influence both the efficiency and specificity of cleavage reaction. We demonstrated that the above-mentioned antisense-added type ribozyme non-specifically cleaved normal *ABL* mRNAs *in vitro* (Kuwabara et al., 1997). This is because a hammerhead ribozyme exerts cleaving ability if it has a binding arm of as small as about 3 nucleotides in length (Hertel et al., 1996; Birikh et al., 1997). Thus, we considered designing a novel maxizyme which forms a catalytically competent structure only in the presence of the junction sequence of L6 *BCR-ABL* (b2a2) mRNA.

The maxizyme of the invention may be produced by chemically synthesizing RNA with a DNA/RNA synthesizer (Model 394; Applied Biosystems, Division of Perkin Elmer Co. (ABI), Foster City, CA), and subjecting the synthesized RNA to deprotection, desalting and purification by PAGE.

The cleaving activity and substrate-specificity of the maxizyme may be assayed as follows. Two substrate RNAs, namely abnormal *BCR-ABL* mRNA (120 mer) and normal *ABL* mRNA (92 mer) are labeled with radioisotope (³²P). These substrate RNAs are mixed in Tris-HCl (pH 8.0) buffer together with a maxizyme and magnesium ion and reacted at 37ºC. After the reaction, the reaction solution is separated by PAGE to detect the presence and absence of cleavage with BAS2000 image analyzer, thereby assaying specific cleavage of *BCR-ABL* mRNA without cleaving *ABL* mRNA.

As will be described later in Examples 1 and 3, when the maxizyme was actually synthesized and assayed for its substrate-specificity, non-specific cleavage did not occur in normal mRNA *in vitro*. The cleavage efficiency of the maxizyme assayed was higher than those of antisense-added type hammerhead ribozymes reported to date or than those of other minizymes.

### 3. Application of a dimeric minizyme to gene therapy

Hereinafter, the obtained maxizyme will be described as to the establishment of its application to gene therapy and as to its assay *in vivo*.

There are two methods for expressing a ribozyme *in vivo*: a method in which a synthesized ribozyme is externally encapsulated with a cationic lipid membrane or the like and then introduced into a cell (Malone, RW. Felgner, PL., Vermn, IM (1989) Proc. Natl. Acad. Sci., USA 86, 6077), and a method in which a ribozyme is introduced into and expressed in a cell as vector DNA (by using a viral vector or the like) (Friedmann. T., Roblin. R. (1972) Science 175, 949). A dimeric minizyme was examined for use in the latter method in which the stability of the promoter and transcript used needs to be considered.

An expression vector containing a DNA coding for a ribozyme of the invention may be produced by connecting promoter and terminator sequences of RNA polymerase III with the maxizyme sequences (tRNA^{Val}-MzL, tRNA^{Val}-MzR)-, and embedding the resultant in a vector such as pUC19 (Takara), pGREEN LANTERN (produced by Life-Tech Oriental) and pHaMDR (HUMAN GENE THERAPY 6:905-915 (July 1995)).

The expression vector produced as described above may be introduced into a cell by the following methods.

### (i) Lipofection method

Since the charge of the surface of a cell is negative, a complex of a vector of interest (double-stranded circular DNA plasmid) and a cationic lipid (lipofection reagent (such as Lipofectin)) is prepared to be introduced into a cell.

### (ii) Viral vector method

This method is more efficient than the method of (i). Among the genetic information of a virus, only the part that is necessary for gene expression is left, into which sequences with a therapeutic effect (DNA sequences of tRNA^{Val}-MzL and tRNA^{Val}-MzR) are incorporated. This vector is introduced into DNA of the target cell utilizing the function of the virus.

The maxizyme contained in the vector sequence is added with the promoter sequence of RNA polymerase III (DNA sequences of tRNA^{Val}-MzL and tRNA^{Val}-MzR). Due to the function of RNA polymeraze III which is natively active in cells, the RNA sequences with a therapeutic effect (tRNA^{Val}-MzL and tRNA^{Val}-MzR) are transcribed, whereby a ribozyme is expressed at high level.

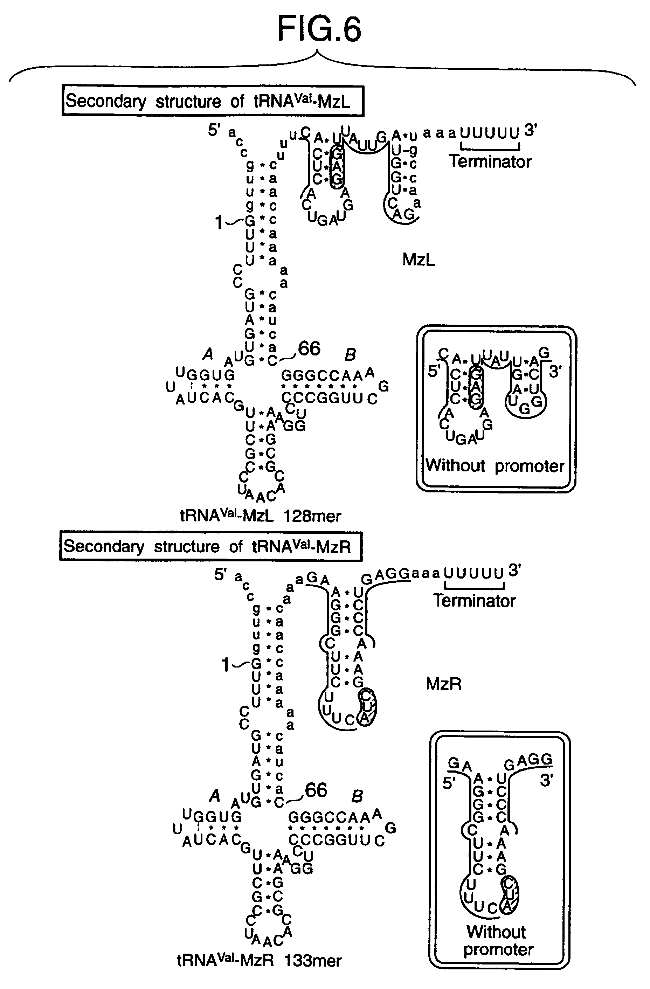

In order to express the ribozyme in the cell at high level, the promoter sequence need to be present upstream of the ribozyme sequence. In employing the expression system of polIII (Geiduschek, EP., Tocchini-Valentin, GP. Transcription by RNA polymerase III, Anun. Rev. Biochem. 57, 873), tRNA^{val} sequence is added as an extra sequence (a promoter sequence other than the ribozyme moiety). An expression vector of the maxizyme was actually constructed as will be described later in Example 2. The ribozyme components expressed from this vector is shown in Figure 6, in which each of the maxizyme sequences is connected downstream of the tRNA^{Val} sequence via a short chain linker. With reference to this figure, the tRNA^{Val} sequence may seem to constitute a significant steric hindrance to the maxizyme. The dimeric structure of the maxizyme is formed to exert the cleaving activity only after the MzL and MzR sequences (MzL: minizyme *left*, MzR: minizyme *right*, which are the two components underlined in Figure 6 forming the dimeric maxizyme (see the active dimer shown in Figure 5)) form base pairs at the stem II region. Since each of the MzL and MzR sequences were preceded by the extra tRNA^{val} sequence with a length of more than 5 times the length of the MzL and MzR sequences, it is possible that a dimer may not be formed due to steric hindrance.

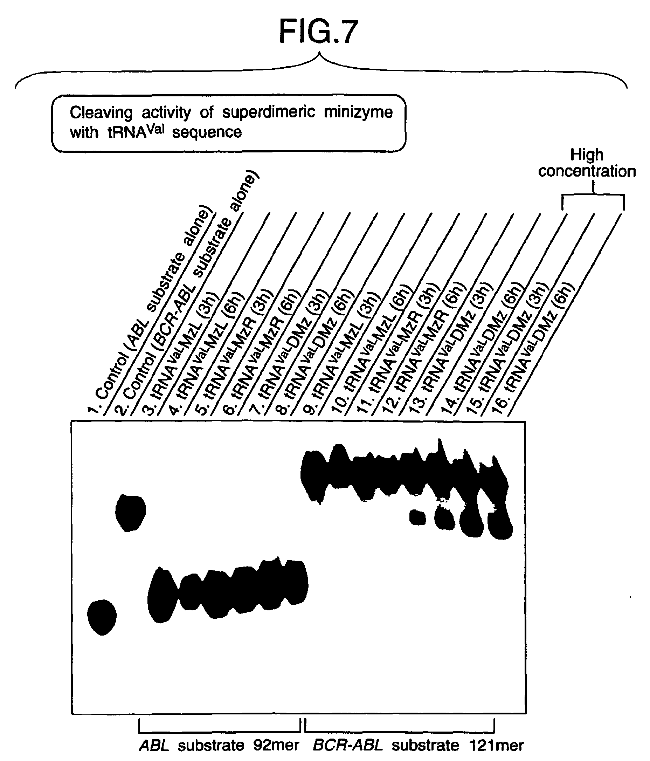

The maxizyme with the tRNA^{Val} sequence and the maxizyme with no extra sequence were assayed *in vitro* for their cleaving activities and substrate-specificities (Example 3). As substrates, *BCR-ABL* chimeric mRNA (121 mer) and *ABL* mRNA (97 mer) as normal mRNA for comparison were prepared and reacted for cleavage with 50 mM Tris-HCl (pH 8.0) and 25 mM MgCl₂ at 37ºC. The results are shown in Figure 7. Surprisingly, cleaving activity was clearly observed for the maxizyme with the tRNA^{val} sequence. In addition, the maxizyme with the tRNA^{val} sequence did not cause any non-specific cleavage in the normal *ABL* mRNA, proving its very high specificity to the *BCR-ABL* chimeric mRNA. Cleavage did not occur in the *BCR-ABL* mRNA substrate because the active dimeric structure was not formed with only one of the maxizyme-forming components, tRNA^{val}-MzL or tRNA^{val}-MzR.

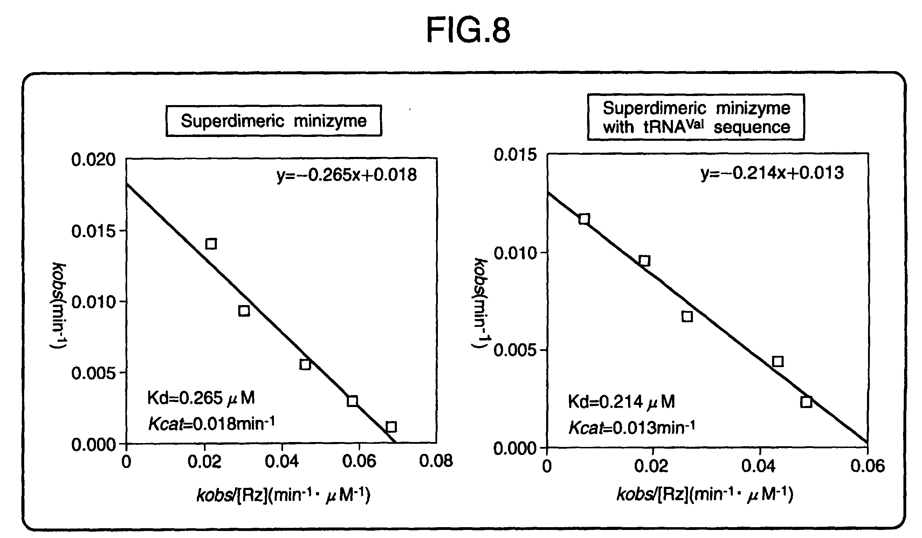

Detailed kinetic analysis of the reaction was performed using a shorter substrate to compare the cleavage efficiency of the maxizyme having the tRNA^{val} sequence with that of the maxizyme with no extra sequence. The results are shown in Figure 8 from which we reached a very interesting conclusion. The k_{cat} and k_{d(app)} values of both maxizymes turned out to be almost the same, surprisingly enough. The tRNA^{val} sequence for enhancing the stability of the maxizyme against RNase *in vivo* was found to have no influence on the cleavage reaction of the maxizyme. To repeat once more, the maxizyme with the tRNA^{val} sequence has very high substrate-specificity and cleaving activity comparative to the maxizyme with no extra sequence.

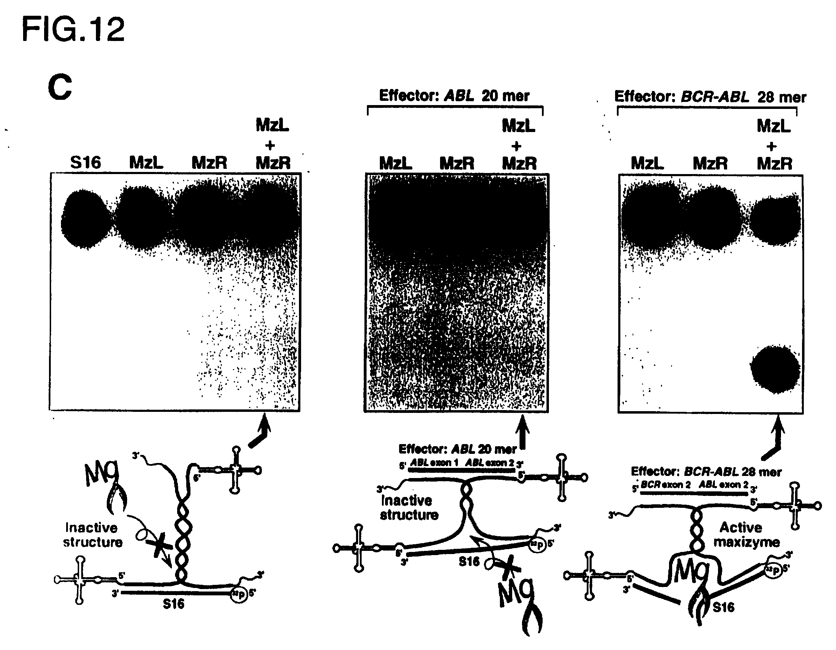

Since we were interested in specific cleavage of b2a2 mRNA, we compared a conventional hammerhead ribozyme (wtRz), two antisense-added type hammerhead ribozymes (asRz52 and asRz81; Pachuk et al., 1994; James et al., 1996) and our novel maxizyme as to their specificities and catalytic activities in cultured cells with respect to cleavage of L6 *BCR-ABL* chimeric (b2a2) mRNA (Example 10). As a result, we found that the activity of our novel maxizyme could be controlled allosterically not only *in vitro* but also in cultured cells in the presence of the junction sequence in L6 *BCR-ABL* mRNA (Figures 12 and 18-21). Specific reduction of the p210^{*BCR-ABL*} protein, as a result of the maxizyme's cleaving activity, led to the cleavage of inactive procaspase-3 to yield active caspase-3, with resultant apoptosis of BaF3/p210^{*BCR-ABL*} cells. Similarly, BV173 cells from a leukemic patient, not normal cells, underwent apoptosis in response to the maxizyme. In contrast, conventional ribozymes caused apoptosis nonspecifically in both BaF3/p210^{*BCR-ABL*} and H9 cells. To the best of our knowledge, this is the first demonstration of an artificially created ribozyme that is under perfect allosteric control not only *in vitro* but also in cultured cells. The novel maxizymes, whose activity can be controlled allosterically by sensor arms that specifically recognize abnormal mRNAs should be powerful tools for disruption of abnormal chimeric targets and might provide the basis for future gene therapy for the treatment of CML.

A maxizyme, which may contain linker and promoter sequences upstream of and a terminator sequence downstream from the ribozyme sequence, may be produced by transcribing a DNA coding for the above sequence to RNA as a template using T7-type enzyme.

A template DNA is prepared by adding a promoter sequence of T7 RNA polymerase before each of the DNA sequences of tRNA^{Val}-MzL and tRNA^{Val}-MzR. This template DNA is mixed with T7 RNA polymerase reaction mixture (buffer, enzyme, NTPs), reacted at 37ºC for 2-4 hours and subjected to purification by PAGE.

The maxizyme of the invention may be used to specifically cleave L6 (b2a2) chimeric mRNA which is causative of chronic myelocytic leukemia. One example of such a method will be described. The maxizyme has two substrate-binding regions. One region binds to a junction site specific to L6 (b2a2) chimeric mRNA while the other region specifically cleaves a cleavable GUC triplet at a remote site (45 residues away) in the presence of magnesium ion. As a result, expression of p210^{*BCR-ABL*} from *BCR-ABL* mRNA can be inhibited without influencing the normal mRNA at all.

The maxizyme of the invention may be used as a medicament, particularly for preventing and/or treating diseases caused by Philadelphia chromosome abnormality.

A vector incorporating a DNA sequence of the maxizyme containing a promoter sequence of RNA polymerase (such as RNA polymerase III) is prepared *in vivo* for introduction. Cells expressing p210^{*BCR-ABL*} are taken from a CML patient, into which the vector is introduced, and cultured. The cultured cells are returned *in vivo*.

As an alternative to the vector to be introduced, the maxizyme RNA may be chemically modified so as to gain resistance to RNase *in vivo*, and introduced into cells, for example, by using a carrier (e.g., cationic lipid, liposome, etc.) or the like.

This specification includes part or all of the contents disclosed in the specifications and/or drawings of Japanese Patent Application Nos. 10-60969 and 10-311098 which are priority documents of the present invention.

### BRIEF DESCRIPTION OF THE DRAWINGS

Figure 1 shows a secondary structure of a hammerhead ribozyme.
Figure 2 shows a process of constructing a dimeric minizyme.
Figure 3 shows a secondary structure of a heterodimeric minizyme.
Figure 4 shows structures and actions of a dimeric minizyme and a maxizyme with *BCR-ABL* chimeric mRNA as a target. In the sturcture of the maxizyme shown at right in Figure 4, the Ys form a stem together and X recognize a specific sequence in the target RNA.
Figure 5 shows a sequence near a junction of *BCR-ABL* chimeric mRNA and normal *ABL* mRNA, as well as the secondary structures of an active maxizyme and an inactive maxizyme.
Figure 6 shows a secondary structure of a maxizyme added with tRNA^{val} sequence. The tRNA^{val} sequence was added such that the secondary structure of the MzL or MzR sequence is as stable as possible, effecting least disruption in the secondary sequence of the case when it consists only of the sequence (framed) (the sequence of the maxizyme is underlined).
Figure 7 shows cleaving activities of maxizymes with and without the tRNA^{val} sequence, targetting *BCR-ABL* chimeric mRNA.
Figure 8 shows rate parameters of maxizymes with and without tRNA^{val} sequence, targeting *BCR-ABL* chimeric mRNA.
Figure 9 shows *BCR-ABL* translocations and fusion mRNAs. The two types of chromosomal translocations [K28-type (upper panel) and L6-type (lower panel)] that are associated with chronic myelogenous leukemia and the corresponding fusion mRNAs are depicted. White boxes represent *BCR* exons and black boxes represent *ABL* exon 2. Dotted lines connecting the *BCR* and *ABL* exons indicate selective splicing pathways. In the L6 b2a2 mRNA, which results from L6 translocations, as well as from some K28 translocations, there are no triplet sequences near the *BCR-ABL* junction that are potentially cleavable by hammerhead ribozymes. A GUC triplet, which is generally the triplet most susceptible to cleavage by hammerhead ribozymes, is located 45 nucleotides from the junction. If such a triplet is selected as the site of cleavage by a ribozyme, the normal *ABL* mRNA that shares a part of the abnormal *BCR-ABL* RNA sequence would also be cleaved by the ribozyme, resulting in damage to the host cells (bottom panel). "nts" refers to Nucleotides.
Figure 10 shows nucleotide sequences of conventional hammerhead and antisense-added type ribozymes. The sequence of L6 *BCR-ABL* mRNA near the junction is enlarged. The sites of cleavage by antisense-added type ribozymes (asRz81 and asRz52) and by the control ribozyme (wtRz) are shown. The site of cleavage by the maxizyme is the same as that by wtRz and the recognition site for the maxizyme is indicated with an arrow.
Figure 11 shows secondary structures of the active and inactive maxizymes. In order to achieve high substrate-specificity, the maxizyme should be in an active conformation only in the presence of the abnormal *BCR-ABL* junction (upper panel), while the conformation should remain inactive in the presence of normal *ABL* mRNA or in the absence of the *BCR-ABL* junction (lower panel). MzL and MzR should allow such conformational change to occur, depending on the presence or absence of the abnormal b2a2 mRNAs.
Figure 12 shows allosteric control of the activity of the maxizyme *in vitro*. The specificity of maxizyme-mediated cleavage was examined by incubating tRNA^{Val}-driven component(s) with the 5'-³²P-labeled short 16-mer substrate (S16) in the presence of an allosteric effector molecule [namely, either a short 20-mer normal *ABL* sequence (20 mer *ABL*) or a short 28-mer *BCR-ABL* sequence (28 mer *BCR-ABL*)]. MzL and/or MzR were incubated at 0.1 µM with 2 nM 5'-³²P-labeled substrate (S16). When the effector, 20-mer *ABL* or 28-mer *BCR-ABL*, was used, the concentration thereof was 1 µM.
Figure 13 shows an assay system for measuring activities of tRNA^{Val}-enzymes in HeLa cells.
Figure 14 shows the effects of tRNA^{Val}-enzymes on the chimeric *BCR-ABL*-luciferase and *ABL*-luciferase genes. Luciferase activity was normalized by reference to the efficiency of transfection which was determined by monitoring activity of a co-transfected gene for β-galactosidase (see "Experimental Procedures" in Example 7)
Figure 15 shows schematic representation of the dependence on IL-3 of BaF3 cells and transduced BaF3 cells that expressed human L6 *BCR-ABL* mRNA.
Figure 16 shows time course of transport to the cytoplasm of the MzL transcript, where N and C represent nuclear fraction and cytoplasmic fraction, respectively.
Figure 17 shows the steady-state levels of expressed tRNA^{Val}-enzymes and localization thereof, where N and C represent nuclear fraction and cytoplasmic fraction, respectively.
Figure 18 shows measurements of viability of tRNA^{Val}-enzyme-transduced BaF3/p210^{*BCR-ABL*} cells and H9 cells. The viability of BV173 cells expressing tRNA^{Val}-enzymes is also shown.
Figure 19 shows morphology of tRNA^{Val}-enzyme-transduced BaF3/p210^{*BCR-ABL*} cells and H9 cells.
Figure 20 shows results of direct detection of the products of cleavage of L6 *BCR-ABL* mRNA in BaF3/p210^{*BCR-ABL*} cells by Northern blot analysis.
Figure 21 shows the results of immunoblot analysis performed using antibody αCPP32, which recognizes the 32-kDa precursor to caspase-3 (procaspase-3) and caspase-3 itself. Cleavage of inactive procaspase-3 yielded active caspase-3 upon specific depletion of p210^{*BCR-ABL*} protein by the maxizyme.
Figure 22 is a picture showing a mouse injected with tumor cells without the maxizyme (control; Mz(-)) and a mouse injected with tumor cells with the maxizyme (maxizyme; Mz(+)), prior to dissection.
Figure 23 is a picture showing spleens of the mouse injected with tumor cells without the maxizyme (control; Mz(-)) and the mouse injected with tumor cells with the maxizyme (maxizyme; Mz(+)).
Figure 24 is a picture showing lymph nodes around thymi of the mouse injected with tumor cells without the maxizyme (control; Mz(-)) and the mouse injected with tumor cells with the maxizyme (maxizyme; Mz(+)).
Figure 25 is a picture showing bone marrows of the mouse injected with tumor cells without the maxizyme (control; Mz(-)) and the mouse injected with tumor cells with the maxizyme (maxizyme; Mz(+)).

### BEST MODES FOR CARRYING OUT THE INVENTION

The present invention will be described in more detail by way of following examples. The scope of the invention is not limited by these examples.

### [Example 1] Synthesis of maxizyme

A CPG column immobilized with a carrier with protected Si groups was used repeating detritylation, coupling, capping and oxidation according to the maxizyme sequences shown in SEQ ID NOS: 1 and 2 to chemically synthesize RNA with a DNA/RNA synthesizer (Model 394; Applied Biosystems, Foster City, CA). After the synthesis, the RNA was eluted from the column with NH₃OH/EtOH, and subjected to deprotection and desalting. The RNA with a chain length of interest was finally isolated by 20% modified PAGE.

### [Example 2] Production of maxizyme with added tRNA^{val} sequence

Double-stranded DNA was prepared by adding the promoter sequence 5'TAATACGACTCACTATA3' (SEQ ID NO: 4) of T7 RNA polymerase and GGG prior to the DNA sequences of tRNA^{val}-MzL and tRNA^{val}-MzR (tRNA^{val}-MzL and tRNA^{val}-MzR sequences as shown in Figure 6).

Using these DNAs as templates, T7 RNA polymerase buffer, T7 RNA polymerase, the template DNA and NTP were placed in a test tube to allow transcription reaction at 37ºC for 2-4 hours. After the reaction, the RNA with the chain length of interest was isolated and purified by 5-8% modified PAGE.

### [Example 3] Assay (in vitro test) for cleaving activities and substrate-specificities of maxizymes with and without the added tRNA^{val} sequence

The *BCR-ABL* substrate containing a junction of *BCR-ABL* and the *ABL* substrate containing a junction of normal *ABL* exon 1-*ABL* exon 2 for comparison were prepared. These substrates were 5'-labeled with radioisotope ³²P, and mixed with the ribozyme as follows (in test tubes).
50 mM Tris-HCl (pH 8.0)
25 mM MgCl₂
1 µM maxizyme (or tRNA^{val}-added type maxizyme)
2 µM [α-³²P]labeled substrate (*BCR-ABL* substrate or *ABL* substrate)

After reacting at 37ºC for 60 minutes, the reaction product (a cleaved product with a shorter length) was detected by 8-20% modified PAGE. Since the maxizyme cleaves only the *BCR-ABL* substrate, cleaved product was not detected in the *ABL* substrate. A cleaved product was detected only in the presence of the *BCR-ABL* substrate (Figure 7).

### [Example 4] Kinetic analysis of reactions of maxizymes with and without the added tRNA^{Val} sequence

In order to facilitate the kinetic analysis, S16 (16-mer RNA containing a GUC triplet) and a 20-mer pseudosubstrate containing the sequence near the *BCR-ABL* junction site, substrates of shorter length, were prepared. S16 was labeled with ³²P, and mixed with excessive (single turnover) enzyme (maxizyme), 25 mM MgCl₂ and 50 mM Tris-HCl (pH 8.0) for cleavage reaction at 37ºC. The initial rate was observed and plotted as Eadie-Hofstee plot to detect the rate constants k_{cat} and k_{d(app)}. The results are shown in Figure 8.

The results indicated that the rate parameters of both maxizymes with and without the added tRNA^{Val} sequence were almost the same. This demonstrates that the tRNA^{Val} sequence does not bring any disadvantage in forming an active dimeric structure and can highly be expected for application in cells.

### [Example 5] Expression of maxizyme with the added tRNA^{Val} sequence

For HeLa cell (obtained from the National Institute of Infectious Diseases), tRNA^{Val}-MzL/pUCdt and tRNA^{Val}-MzR/pUCdt vectors were prepared by insertion of DNA sequences of tRNA^{Val}-MzL and tRNA^{Val}-MzR. The vectors were introduced into the cell and subjected to Northern hybridization experiment, whereby both tRNA^{Val}-MzL and tRNA^{Val}-MzR RNAs were confirmed to be stably expressed at high levels.

Briefly, plasmid pUCdt, obtained by incorporating tRNA^{Val} into the commercially available pUC119, was cleaved with restriction enzymes Csp45I and SalI. To this plasmid, a Csp45I-SalI fragment of the DNA sequences of tRNA^{Val}-MzL and tRNA^{Val}-MzR were connected with DNA ligase, thereby producing tRNA^{Val}-MzL/pUCdt and tRNA^{Val}-MzR/pUCdt vectors. Using lipofectin (commercially available from Gibco-BRL), the vectors were transfected into cells (lipofection method). Total RNA expressed in the cells was extracted therefrom 36 hours after culturing the cells. The RNAs extracted from the cells were subjected to Northern hybridization to detect expression of minizyme in the cells using DNA sequences complementary to MzL and MzR sequences.

### [Example 6] Specific design of a novel maxizyme under the control of a human tRNA^{Val}-promoter, and in vitro demonstration of the allosteric control of its activity by the junction sequence of BCR-ABL mRNA

For application of a maxizyme to gene therapy for treating CML, it is important that the maxizyme be expressed *in vivo* constitutively and under the control of a strong promoter. Each monomeric unit was incorporated downstream of the sequence of a human tRNA^{Val}-promoter (Baier et al., 1994; Yu et al., 1995; Kawasaki et al, 1996, 1998; Bertrand et al., 1997) which is recognized by RNA polymerase III (Geiduschek and Tocchini-Valentini, 1988; Perriman and de Feyter, 1997)-to create MzL (maxizyme *left*) and MzR (maxizyme *right*; Figure 6). Higher-level expression under the control of the polymerase III promoter would clearly be advantageous if maxizymes are to be used as therapeutic agents and such an expression would also increase the likelihood of dimerization.

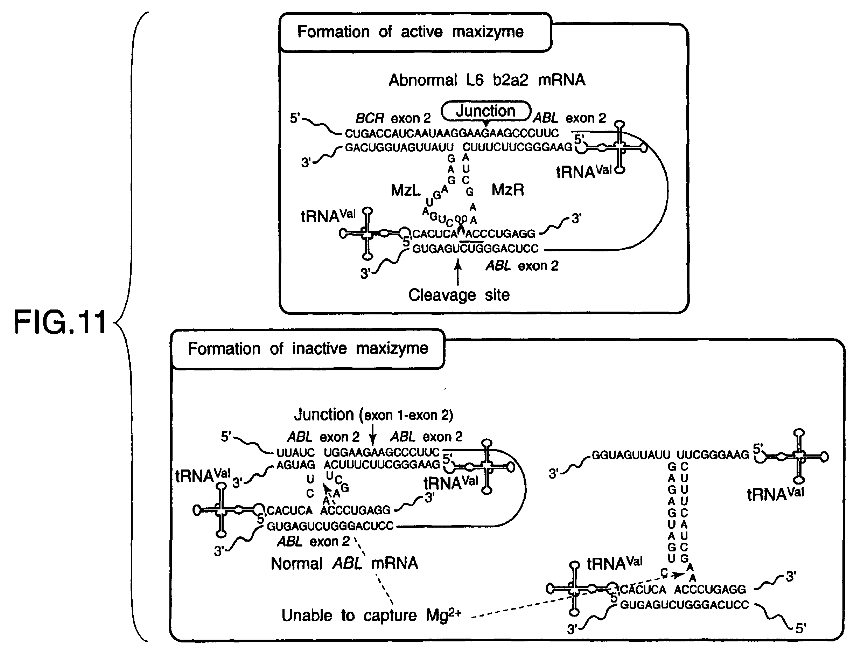

In order to achieve high substrate-specificity, our maxizyme should adopt an active conformation only in the presence of the abnormal *BCR-ABL* junction (Figure 11, top panel), while the conformation should remain inactive in the presence of the normal *ABL* mRNA and in the absence of the abnormal *BCR-ABL* junction (Figure 11, bottom panel). The specifically designed sequences, which are shown in Figure 11 (note that the length and sequence of the sensor arm and those of common stem II are variable), should permit such conformational changes depending on the presence or absence of the abnormal b2a2 mRNA. This phenomenon may resemble the changes in conformation of allosteric proteinaceous enzymes in response to their effector molecules. In order to compare the activity and specificity of our maxizyme with that of a conventional wild-type ribozyme (wtRz) targeted to the same cleavage site, and with those of conventional antisense-added type ribozymes (asRz52 and asRz81; Figure 10), we embedded the latter two types of ribozyme in the 3'portion of the gene for tRNA^{Val}.

In order to prove *in vitro* that conformational changes depended on the presence or absence of the abnormal L6 b2a2 mRNA, we prepared a short 16-nucleotide (nt) *BCR-ABL* substrate (S16) that corresponded to the target site indicated by capital letters in the upper panel of Figure 11. The specificity was tested by incubating the *in vitro* transcribed maxizyme with the 5'-³²P-labeled short 16-mer substrate (S16) in the presence and in the absence of either a 20-mer normal *ABL* effector molecule or a 28-mer L6 *BCR-ABL* effector molecule. These effector molecules corresponded respectively to the sequences indicated by capital letters in the normal *ABL* mRNA on the left in the lower panel of Figure 11 and in the abnormal L6 b2a2 mRNA in the upper panel in Figure 11.

### Experimental Procedure

### Construction of plasmids for expression of tRNA-embedded enzymes

Chemically synthesized oligonucleotides encoding each enzyme (MzL, MzR, wtRz, asRz52 and asRz81) and the polymerase III termination sequence (Geiduschek and Tocchini-Valentini, 1988) were converted to double-stranded sequences by PCR. After digestion with *Csp*45I and *Sal*I, each appropriate fragment was cloned downstream of the tRNA^{Val} promoter of pV (which contained a chemically synthesized promoter of a human gene for tRNA^{Val} between the *Eco*RI and *Sal*I sites of the pMX puro vector; Kitamura et al., 1995). The sequences of the constructs were confirmed by direct sequencing.

### Assays for the activities of the maxizyme and ribozymes in vitro

Assays for the activities of the maxizyme and ribozymes were performed, in 25 mM MgCl₂ and 50 mM Tris-HCl (pH 8.0), under enzyme-saturated (single-turnover) conditions at 35ºC, with incubation for 60 minutes (Figure 12). The substrates were labeled with [γ-³²P]-ATP by T4 polynucleotide kinase (Takara Shuzo, Kyoto, Japan). Each enzyme was-incubated at 1 µM concentration with 2 nM 5'-³²P-labeled S16 (see "Results"). Reactions were initiated by addition of MgCl₂ to a buffered solution that contained each enzyme with the substrate, and each resultant mixture was then incubated at 37ºC. Finally, reaction mixtures were subjected to electrophoresis on an 8% polyacrylamide/7 M urea gel.

### Results

The 28-mer L6 *BCR-ABL* effector molecule corresponding to the junction sequence in b2a2 mRNA acted *in trans*. This molecule should be annealed as being recognized by the sensor arms of MzL and MzR and should serve to direct formation of an active dimer. The other recognition arms of the maxizyme should recognize the cleavable triplet in the short 16-mer *BCR*-*ABL* substrate RNA and specific cleavage should occur (Figure 12, right). No cleavage products of the substrate were detected in the absence of the *BCR-ABL* junction or in the presence of the normal *ABL* sequence (effector molecule), demonstrating the expected high substrate-specificity of the maxizyme.

Since MzL or MzR by itself, in the presence and in the absence of effector molecules, did not have any cleaving activity, the active species was clearly the dimeric maxizyme shown at the bottom right in Figure 12, which was involved in a tetramolecular interaction. In principle, the bimolecular interactions of the conventional ribozyme are more favorable than tetramolecular interactions. Nevertheless, the cleaving activity of the maxizyme was nearly identical to that of the hammerhead ribozyme (wtRz; data not shown). The activity of the maxizyme was also demonstrated to be greater than that of the conventional hammerhead ribozyme in cultured cells, as described in the following section. Similar results were obtained when the effector sequence was connected with the cleavage sequence, as depicted in Figure 11, with the maxizyme involved in trimolecular interactions (data not shown). The results shown in Figure 12 prove that the maxizyme was subjected to complete allosteric control *in vitro*, in accord with the conformational changes (depicted in Figure 11) that should occur in response to the effector molecule (the *BCR-ABL* junction sequence) that was added *in trans*. Furthermore, the above-mentioned results confirm that the tRNA^{Val}-portion did not interfere with the allosteric control.

### [Example 7] Comparison of the intracellular activities of the maxizyme and those of conventional hammerhead ribozymes in mammalian cells

we next examined the action of the maxizyme in mammalian cells using a reporter construct. To evaluate the intracellular activity of the maxizyme, we co-transfected HeLa cells with expression plasmids that encoded an appropriate enzyme unit(s) under the control of the human tRNA^{Val}-promoter, together with a target gene-expressing plasmid pB2A2-luc (or p*ABL*-luc) that encoded a chimeric target *BCR-ABL* (or *ABL* alone) sequence and a gene for luciferase. The junction-expressing plasmid pB2A2-luc contained a sequence of 300 nts that encompassed the *BCR-ABL* junction. The plasmid, p*ABL*-luc, contained a sequence of 300 nts that encompassed the junction between exon 1 and exon 2 of the normal *ABL* mRNA. After transient expression of both genes, in individual cell lysate, we estimated the intracellular activity of each enzyme by measuring the luciferase activity.

### Experimental Procedure

### Preparation of tRNA^{Val}-enzymes by transcription

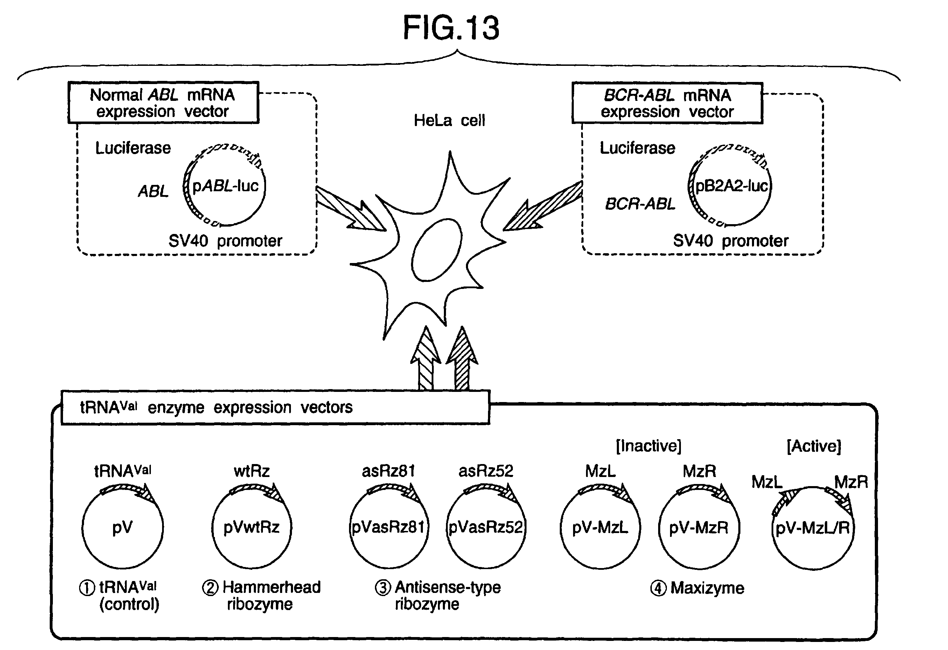

The tRNA^{Val}-enzyme expression vectors shown in Figure 13 were used as DNA templates for PCR for constructing DNA templates for transcription. Primers were synthesized for each template, with the sense strand containing the T7 promoter. T7 transcription *in vitro* and purification were performed as described in a publication (Kuwabara et al., 1996).

### Assays for reporter activity after transient transfection

Luciferase activity was measured with a PicaGene Kit (Toyo-ink, Tokyo, Japan) as described in a publication (Koseki et al., 1998). In order to normalize the effect of transfection with reference to β-galactosidase activity, cells were co-transfected with the pSV-β-galactosidase control vector (Promega, Madison, WI) and then the chemiluminescent signal due to β-galactosidase was quantitated with a luminescent β-galactosidase genetic reporter system (Clontech, Palo Alto, CA) as described (Koseki et al., 1998).

### Results

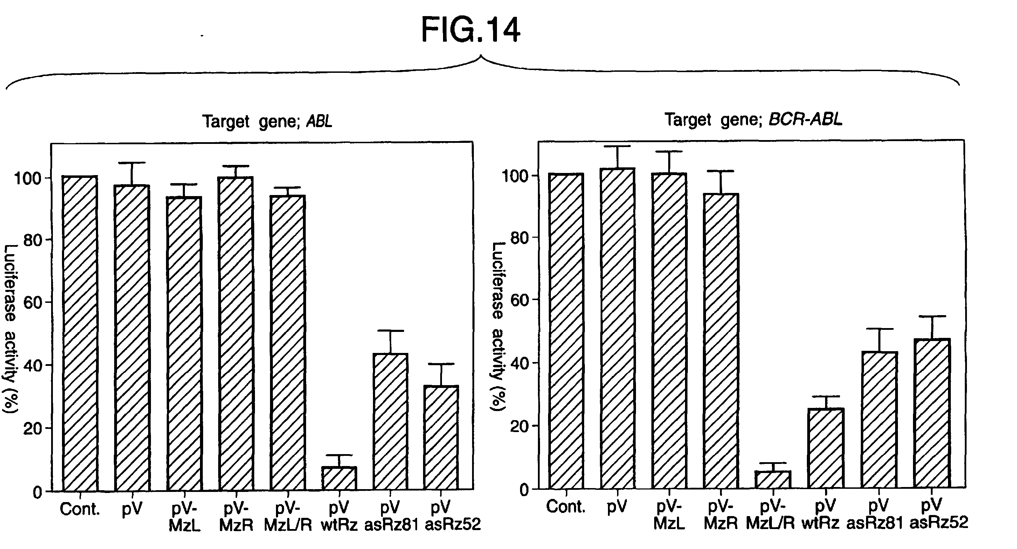

The luciferase activity recorded for the target gene-expressing plasmid (pB2A2-luc or p*ABL*-luc) was taken as 100% (Figure 14). Expression of the tRNA^{Val}-portion (pV) alone had no inhibitory effect. In contrast, the novel maxizyme (pV-MzL/R) was extremely effective in cell culture in suppressing the *BCR*-*ABL*-luciferase gene (>95% inhibition) (Figure 14, right panel), and it had no inhibitory effects on expression of the *ABL*-luciferase gene (Figure 14, left panel), demonstrating the extremely high specificity of the maxizyme. As expected, the conventional hammerhead ribozyme (pVwtRz), targeted to the same site as the maxizyme, suppressed the expression of both the *BCR-ABL*-luciferase gene and the *ABL*-luciferase gene. It is important to note that, despite the original expectations of high specificity, the conventional antisense-added type ribozymes (pVasRz81 and pVasRz52) also suppressed the expression of both the *BCR-ABL*-luciferase gene and the *ABL*-luciferase gene, acting non-specifically, in agreement with our previous findings *in vitro* (Kuwabara et al., 1997). Moreover, the extent of suppression by the conventional (antisense-added type) ribozymes was not as much as that by the maxizyme.

The individual subunits of the maxizyme (MzL and MzR) had no inhibitory effects. Thus, the activity of the maxizyme must have originated from the formation of active heterodimers in the mammalian cells. Moreover, since the maxizyme specifically inhibited the expression of the *BCR-ABL*-luciferase gene without affecting the related *ABL*-luciferase gene that contained a potential site of cleavage by the maxizyme, complete allosteric regulation must be operative in the mammalian cells. To the best of our knowledge, this is the first demonstration of complete allosteric regulation in mammalian cells of the activity of an artificially created enzyme.

### [Example 8] Generation of stable transformants of the BaF3 cell line (BaF3/p210^{BCR-ABL}) that expressed human L6 BCR-ABL mRNA, and of BaF3/p210^{BCR-ABL} and H9 cell lines transduced with the tRNA^{Val}-ribozymes or the tRNA^{Val}-maxizyme

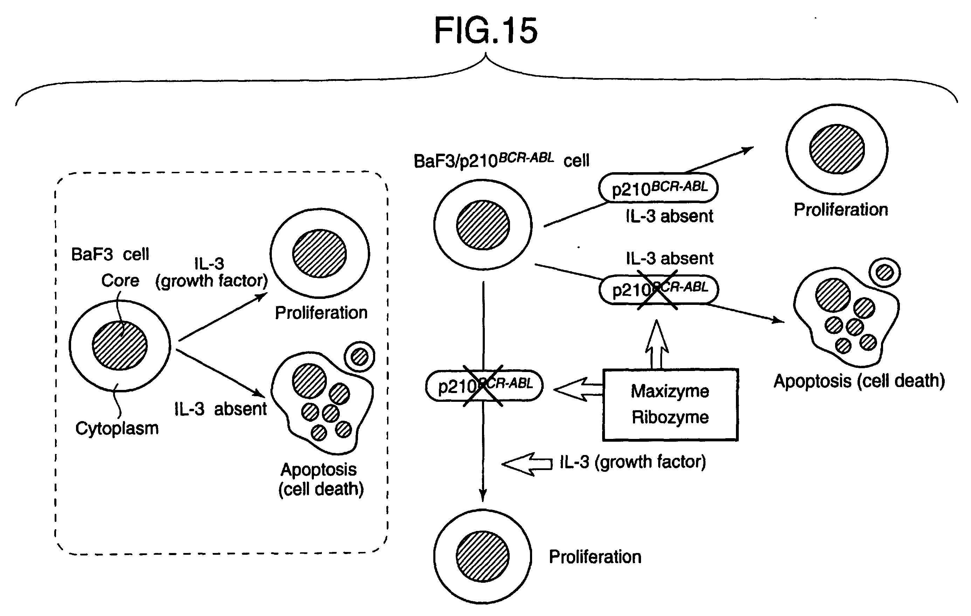

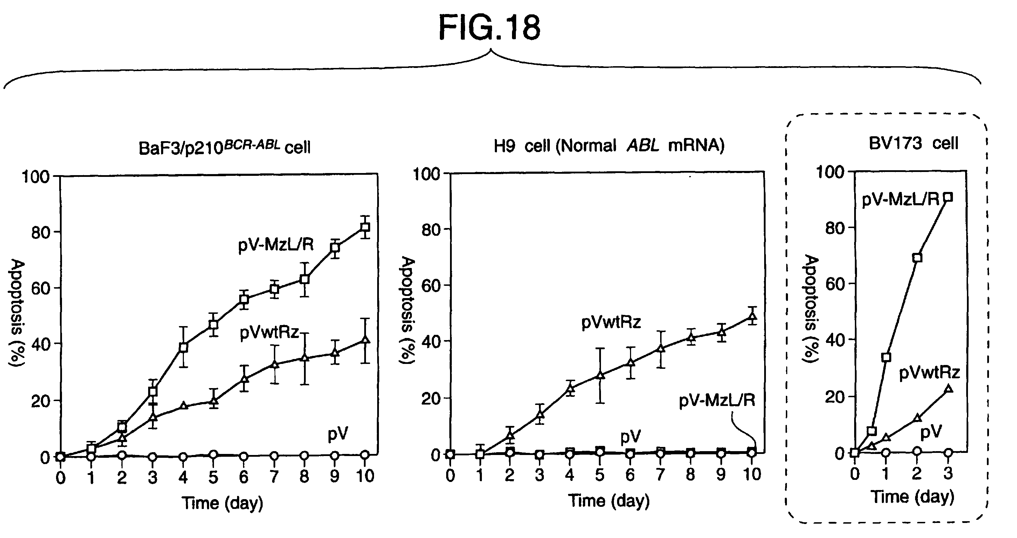

Since the maxizyme had acted efficiently and specifically against the reporter gene construct in HeLa cells (Example 7), we decided to examine the activity of the maxizyme against an endogenous *BCR-ABL* (L6 b2a2 mRNA) target. We established a murine cell line, BaF3/p210^{*BCR-ABL*}, that expressed human L6 b2a2 mRNA constitutively, by integrating a plasmid construct that expressed p210^{*BCR-ABL*} (pMX/p210^{*BCR-ABL*}; p210^{*BCR-ABL*} was generated from human L6 b2a2 mRNA). We should emphasize that this cell line (which expressed L6 b2a2 mRNA) was different from the cell line (BaF3+p210 cells that expressed K28 b3a2 mRNA) used previously by Daley and Baltimore (1988) and Choo et al., (1994).
Although the parental BaF3 cell line is an interleukin-3(IL-3)-dependent hematopoietic cell line (Daley and Baltimore, 1988; left panel of Figure 15), the transformed BaF3/p210^{*BCR-ABL*} were IL-3-independent because of the tyrosine kinase activity of p210^{*BCR-ABL*} and, thus, the latter transformed cells were able to grow in the absence of IL-3 (Figure 15, right). However, if the expression of p210^{*BCR-ABL*} were to be inhibited, BaF3/p210^{*BCR-ABL*} cells should become IL-3-dependent and, in the absence of IL-3, they should undergo apoptosis. Therefore, for the selection of maxizyme- or ribozyme-transduced BaF3/p210^{*BCR-ABL*} cells, we used 10% WEHI-conditioned RPMI medium as a source of IL-3. In the presence of IL-3, BaF3/p210^{*BCR-ABL*} cells were transfected separately with plasmids pV, pVwtRz and pV-MzL/R (Figure 13), all of which encoded a gene resistant to puromycin. In order to generate BaF3/p210^{*BCR-ABL*} cells that had been stably transduced with tRNA^{Val}, wtRz or maxizyme constructs, the medium was replaced, after 24 hours of transfection, with RPMI medium supplemented with 10% FCS and 3 µg/mL puromycin. The transduced cells were cultured for additional 60 hours and then IL-3 was removed from the medium for assays of apoptosis (see below; results shown in Figures 18 to 21).

In order to examine the specificity of the maxizyme, we also used H9 cells originating from human T cells, and also examined the influence on expression of normal *ABL* mRNA as a control. Stably transduced H9 cells that harbored a maxizyme or ribozyme construct were generated using the respective plasmids (described above). The efficiency of transfection was very low, so we generated transduced cells using a cell line of retroviral producer cells (BOSC23 cells). Filtered supernatants of BOSC23 cells, which had been transfected with plasmids pV, pVwtRz or pV-MzL/R, were added to H9 cells. The H9 cells were cultured for 72 hours and then puromycin was added for selection of resistant cells. The various lines of transduced cells allowed us to examine the activity and specificity of the maxizyme and ribozymes against an endogenous target gene (rather than a reporter construct).

### Experimental Procedure

### Construction of the BaF3/p210^{BCR-ABL} cell line

Cells that stably expressed human L6 *BCR-ABL* mRNA were obtained by retroviral infection of BaF3 cells that had been growing in the presence of WEHI-conditioned medium as a source of IL-3. Helper-free retrovirus stocks were produced in BOSC23 cells with the pMX-p210^{*BCR-ABL*} vector, which encoded human L6 *BCR-ABL* mRNA, according to the procedure described by Muller et al (Muller et al, 1991). Retroviral infections of BaF3 cells were performed according to the method of Pendergast et al.
(Pendergast et al., 1993). IL-3 was removed 72 hours after the infection to allow selection for populations that expressed the fusion gene. BaF3/p210^{*BCR-ABL*} cells were maintained in RPMI-1640 medium supplemented with 10% fetal calf serum (FCS; Gibco-BRL, Rockville, MD) and 3 µg/mL puromycin (Gibco-BRL).

### [Example 9] Efficient expression and transport to the cytoplasm of the maxizyme

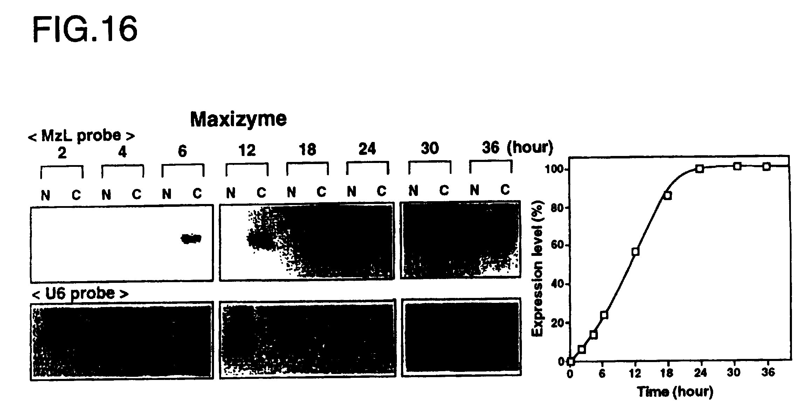

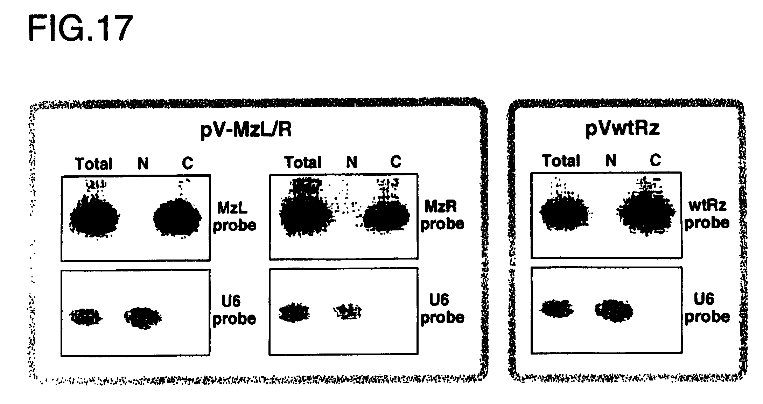

In addition to the level of expression and the half-life of an expressed ribozyme, the co-localization of the ribozyme with its target is obviously an important determinant of the ribozyme's efficiency *in vivo* (Sullenger and Cech, 1993; Eckstein and Lilley, 1996; Bertrand et al., 1997). Therefore, it was essential to determine the intracellular localization of each of our tRNA^{Val}-enzymes. To confirm the expression and relative stability of the maxizyme in BaF3/p210^{*BCR-ABL*} cells, we performed Northern blot analysis (Figure 16 and 17). Total RNA from BaF3/p210^{*BCR-ABL*} cells that had been transfected with the various plasmids was extracted 2, 4, 6, 12, 18, 24, 30 and 36 hours after transfection. Samples of total RNA were also separated into nuclear and cytoplasmic fractions.

### Experimental Procedure

### Northern blot analysis

For the assay for expression of target mRNA and tRNA^{Val}-enzyme transcript in BaF3/p210^{*BCR-ABL*} cells, total RNA was isolated with ISOGEN™ (Nippon Gene Co., Toyama). Cytoplasmic RNA and nuclear RNA were separated according to the method of Huang and Carmichael (Huang and Carmichael, 1996). Thirty µg of total RNA per lane were loaded on an agarose gel (FMC Inc., Rockland, ME), and then bands of RNA were transferred to a Hybond-N™ nylon membrane (Amersham Co., Buckinghamshire, UK). The nylon membrane was probed with synthetic oligonucleeotides, which were complementary to the sequences of MzL, MzR, wtRz and L6 *BCR-ABL* junction (Figure 10) that had been labeled with ³²P by T4 polynucleotide kinase (Takara Shuzo Co., Kyoto, Japan). Prehybridization and hybridization were performed as described by Koseki et al. (Koseki et al., 1998).

### Results

Transcripts of about 130 nucleotides in length, which corresponded in size to MzL were detected (Figure 16). Even initially, MzL transcripts were found in the cytoplasmic fraction and not in the nuclear fraction (Figure 16). The time course of changes in the level of the maxizyme in the cytoplasm is shown in the right panel of Figure 16. MzL was detected within 4 hours and its expression level reached a plateau 24 hours after transfection.

We next estimated the steady-state levels and localization of MzL, MzR and wtRz in BaF3/p210^{*BCR-ABL*} cells that had been stably transduced with the respective maxizyme-encoding and ribozyme-encoding plasmids. Total RNA that had been isolated 3 days after removal of IL-3 (Figure 17), as described in the previous section, was used in this analysis. The result in Figure 17 clearly demonstrate that each tRNA^{Val}-enzyme was expressed at significant levels and the transcripts were obviously stable. Furthermore, all tRNA^{Val}-enzymes were found in cytoplasmic fractions and not to any significant level in nuclear fractions. Analysis of the localization of U6 snRNA, which remains in the nucleus (Terns et al., 1993), was included in these studies as a control (Figures 16 and 17). The similar stabilities and cytoplasmic localization of each tRNA^{Val}-transcript in H9 cells were also confirmed by Northern blot analysis (data not shown).

The finding that both the transiently expressed transcripts (Figure 16) and transcripts in stable transformants (Figure 17) were stable and co-localized with their targets in the cytoplasm serves to emphasize the potential usefulness of our modified tRNA^{Val}-expression system in future gene therapy.

### [Example 10] The activity and specificity of the maxizyme against an endogenous BCR-ABL cellular target

We examined the functional significance of the maxizyme in the regulation of apoptosis. We transfected BaF3 cells that stably expressed L6 *BCR-ABL* (b2a2) mRNA with plasmids that encoded the wild-type ribozyme (pVwtRz), the maxizyme (pV-MzL/R) or the parental vector (pV), and selected cells by exposure to puromycin 24 hours after the transfection. After incubation for 60 hours in the presence of puromycin, poststationary cells were removed by Ficol and puromycin-resistant cells were cultured for various times in a medium free of IL-3. Cell viability was assayed in terms of the ability to exclude trypan blue dye. In addition to BaF3/p210^{*BCR-ABL*} cells, we used H9 cells that expressed normal *ABL* mRNA at high levels as a control.

### Experimental Procedure

### Cell viability and apoptosis

Cell viability was determined by trypan blue exclusion. Apoptosis was determined as described by Reuther et al. (Reuther et al., 1998), and the cells were stained with 10 µg/mL Hoechst33342 (Nippon Gene Co., Toyama) for 15 min to study nuclear morphology. After washing and mounting in 90% glycerol/20 mM Tris (pH 8.0)/0.1% N-propyl gallate, slides were examined using a fluorescent microscope (Nikon, Tokyo).

### Results

As shown in Figure 18, BaF3/p210^{*BCR-ABL*} cells that expressed maxizyme died at an accelerated rate relative to the BaF3/p210^{*BCR-ABL*} (pV) cells transfected with the control plasmid. In the experiment whose results are shown in the figure, for example, only about 20% of maxizyme-transfected BaF3/p210^{*BCR-ABL*} cells remained alive 10 days after withdrawal of IL-3, while nearly 100% of BaF3/p210^{*BCR-ABL*} (pV) cells were alive (Figure 18, left). Moreover, the maxizyme did not kill any H9 cells that expressed normal *ABL* mRNA (Figure 18, middle). This result demonstrates the high specificity for targeting the chimeric *BCR-ABL* gene (see below for direct evidence). In contrast, the conventional hammerhead ribozyme, wtRz, induced apoptosis in both BaF3/p210^{*BCR-ABL*} and H9 cells (Figure 18, left and middle), which is consistent with the observation that wtRz can target the transcripts of both the *BCR-ABL* gene and the normal *ABL* gene in vitro and in culture cells (Figures 12 and 14). Furthermore, the maxizyme greatly caused cell death in BV173 cells, derived from a leukemic patient with a Philadelphia chromosome (obtained from the Institute of Medical Science, the University of Tokyo), compared to cells expressing wild-type ribozyme or the parental vector (Figure 18, right).

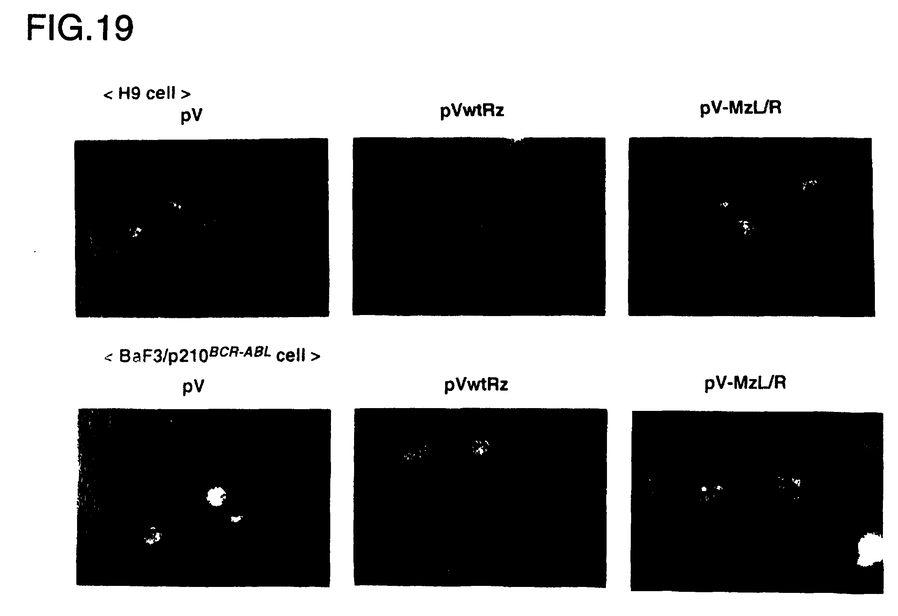

Microscopic examination of dead cells after staining with the DNA-binding fluorochrome Hoechst33342 revealed typical apoptotic morphology, which included condensed chromatin, fragmented nuclei, and shrunken cell size (Figure 19). It was clear that the maxizyme (pV-MzL/R) had caused apoptotic cell death specifically in BaF3/p210^{*BCR-ABL*} cells without affecting normal H9 cells, whereas the ribozyme (pVwtRz) had induced apoptosis in both BaF3/p210^{*BCR-ABL*} and H9 cells. As expected, the expression of the control tRNA^{Val} RNA itself (pV) did not change the morphology of either type of cell. The level of apoptosis induced by the maxizyme was higher than that induced by wtRz in BaF3/p210^{*BCR-ABL*} cells, demonstrating the higher cleaving activity of the maxizyme than that of the conventional ribozyme against the endogenous target.

### [Example 11] Direct evidence for the cleavage of L6 BCR-ABL mRNA and enhanced activation of procaspase-3 by the maxizyme and ribozyme

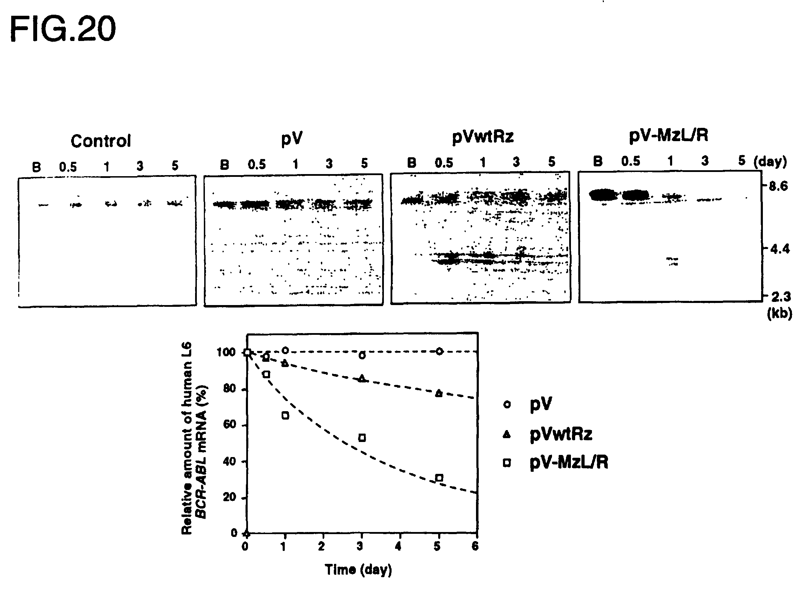

Since the maxizyme and the ribozyme overcame the *BCR-ABL-*mediated inhibition of apoptosis, we tried to detect the anticipated cleavage products directly by Northern blot analysis (Figure 20). Total RNA from tRNA^{Val}-enzyme-transduced BaF3/p210^{*BCR-ABL*} cells was extracted 0.5, 1, 3 and 5 days after the removal of IL-3. The levels of L6 *BCR-ABL* mRNA were determined from the autoradiogram. The length of the cleavage products was exactly as anticipated (about 3 kb). Time courses of the decrease in levels of L6 *BCR-ABL* mRNA in the presence of the tRNA^{Val}-enzymes are shown in the lower panel in Figure 20, in which the basal level of L6 *BCR-ABL* mRNA in the BaF3/p210^{*BCR-ABL*} cells was taken as 100% (the upper panel labeled "Control" in Figure 20 shows the basal level of expression of L6 *BCR-ABL* mRNA at each time interval). No decrease in the level of expressed L6 *BCR-ABL* mRNA was observed in the case of the control tRNA^{Val}RNA (pV). The rate of disappearance of L6 *BCR-ABL* mRNA was clearly more rapid in cells that produced the maxizyme than in those that produced wtRz. The half-life of L6 *BCR-ABL* mRNA was about 3 and about 10 days in cells that produced the maxizyme and wtRz, respectively. The cleavage products of endogenous *BCR-ABL* mRNA generated as a result of the expression of the maxizyme was also confirmed of their presence in BV173 cells (data not shown). Detection of these fragments proved that the maxizyme and the conventional ribozyme were catalytically active and cleaved specifically the target mRNA in cultured cells. Thus, we confirmed that the apoptosis of cells shown in Figures 18 and 19 originated from the cleavage of L6 *BCR-ABL* mRNA by the maxizyme or of L6 *BCR-ABL* and *ABL* mRNAs by the ribozyme, with resultant depletion of p210^{*BCR-ABL*} and/or p145 c-*ABL* proteins in the respective hematopoietic cells. The p145 c-*ABL* protein is a nuclear protein with low intrinsic tyrosine kinase activity, whereas the p210^{*BCR-ABL*} protein is a cytoplasmic, membrane-associated protein with a constitutively high level of tyrosine kinase activity that prolongs the survival of hematopoietic cells by inhibiting apoptosis.

Transduction of the apoptotic signal and execution of apoptosis require the coordinated actions of several aspartate-specific cysteine proteases, known as caspases. An inverse relationship between the *BCR-ABL*-mediated inhibition of apoptosis and the activation of procaspase-3 was recently proven by Dubrez, et al., (1998). Therefore, we investigated whether the maxizyme- (or ribozyme-) mediated apoptotic pathway would indeed involve the activation of procaspase-3 in leukemic cells. We asked whether the specific depletion of p210^{*BCR-ABL*} protein by the maxizyme would lead to the cleavage of inactive procaspase-3 to yield active caspase-3, with resultant apoptosis in BaF3/p210^{*BCR-ABL*} cells. Immunoblot analysis using antibody αCPP32, which recognizes both the 32-kDa inactive precursor of caspase-3 (procaspase-3) and the processed, active protease, caspase-3, enabled us to trace the maturation process. In order to examine the specificity of the maxizyme, we performed a similar study using H9 cells.

### Experimental Procedure

### Western blot analysis

Cell lysates were subjected to SDS-PAGE on a 15% polyacrylamide gel. Rabbit polyclonal αCPP32 antibody (courteously provided by Professor Hong-Gang Wang, University of South Florida, College of Medicine) that recognizes both procaspase-3 and the processed p17 (caspase-3) was used to detect procaspase-3 activation in apoptotic BaF3/p210^{*BCR-ABL*} and H9 cells. The blocking and detection were performed according to the method of Dubrez et al. (Dubrez et al., 1998).

### Results

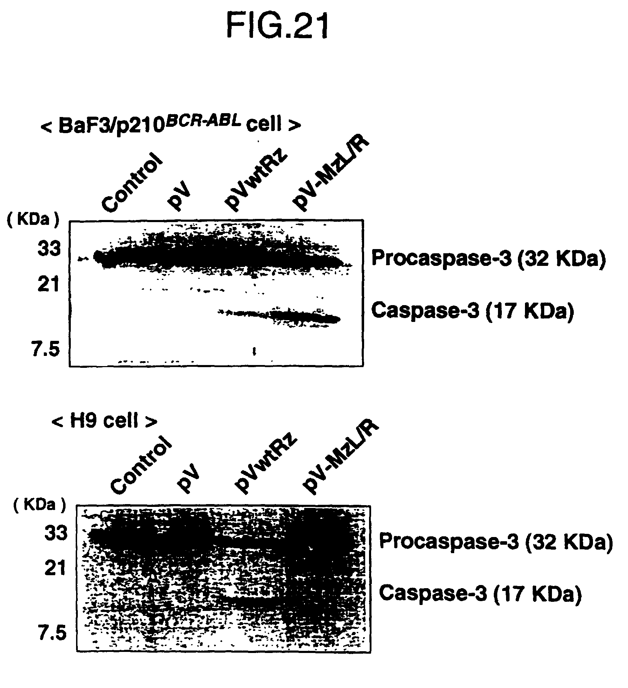

The basal level of procaspase-3 was almost the same in both BaF3/p210^{*BCR-ABL*} and H9 cells (Figure 21). In maxizyme-transduced BaF3/p210^{*BCR-ABL*} cells, the level of procaspase-3 decreased and the level of the p17 active subunit of caspase-3 increased. In stably maxizyme-transduced H9 cells, the level of procaspase-3 remained unchanged. In contrast, expression of the wild-type ribozyme was associated with the processing of procaspase-3 in both BaF3/p210^{*BCR-ABL*} and H9 cells. The rate of conversion of procaspase-3 to caspase-3 in stably maxizyme-transduced BaF3/p210^{*BCR-ABL*} cells was higher than that in wtRz-transduced BaF3/p210^{*BCR-ABL*} cells. These data strengthen our conclusion that the maxizyme induced apoptosis as a result of specific depletion of p210^{*BCR-ABL*} protein, thereby promoting activation of caspase-3 in leukemic cells.

### [Discussion]

We have described herein the first, to our knowledge, novel design of an allosterically modulated RNA catalyst (maxizyme) that selectively cleaves a specific phosphodiester bond. The design was based on a dimeric RNA motif that is catalytically activated by interaction with a specific short sequence (sequence of interest; Figure 1) that is recognized by the sensor arm that are some distance from the active site. The design of the tRNA^{Val}-embedded maxizyme is based on our previous successful attachment of a ribozyme sequence to the 3'-modified side of the tRNA^{Val}-portion of a human tRNA gene, which yielded very active ribozymes with high specificity in cultured cells (Kawasaki et al., 1996, 1998). Although we feared initially that the tRNA^{Val}-portion of the transcript might hinder the dimerization of the tRNA^{Val}-driven RNAs, our analysis indicated that the tRNA^{Val}-portions were located at some distance from each other during dimerization and, thus, they did not interfere with the dimerization process (Kuwabara et al., 1998). The present analysis confirmed the dimerization of the tRNA^{Val}-driven monomer units of the maxizyme. More importantly, the tRNA^{Val}-driven maxizyme underwent a conformational change in response to allosteric effectors (Figure 5), not only *in vitro* (Figure 12) but also in various kinds of cultured cells that included cells from a patient with leukemia.

Although creation of artificial allosteric enzymes is currently of a great interest (Porta and Lizardi, 1995; Tang and Breaker, 1997a, 1997b), to the best of our knowledge, no such enzyme has yet been tested in animals or in cultured cells. Our novel maxizyme cleaved L6 *BCR-ABL* mRNA specifically without damaging the normal *ABL* mRNA in cultured cells, providing the first example of successful allosteric control of the activity of an artificially created allosteric enzyme. In past efforts to destroy L6 *BCR-ABL* mRNA by antisense molecules, it was difficult to demonstrate specificity. Since both the p210^{*BCR-ABL*} chimeric protein and the p145 c-*ABL* protein are negative regulators of apoptosis (Chapman et al., 1994; Laneuville et al., 1994; Spooncer et al., 1994; Bedi et al., 1994, 1995; McGahon, et al., 1994, 1995; Dubrez et al., 1998), antisense molecules with low specificity can induce apoptosis in leukemic cells by inhibiting expression of normal *ABL* mRNA in addition to blocking the *BCR-ABL* pathway. In fact, it was reported in recent publications that no decrease in the level of p210^{*BCR-ABL*} protein was observed in apoptotic cells that had been treated with antisense molecules, and nonspecific inhibition by such antisense oligonucleotides resulted from their non-antisense effects (O'Brien et al., 1994; Maekawa et al., 1995; Mahon et al., 1995; Smetsers et al., 1995, 1997; Vaerman et al., 1995, 1997). we also realized that the introduction of modifications into antisense molecules, such as the introduction of phosphorothioate moieties, causes cell death in a manner that is not sequence-specific. In our hands, even unmodified antisense DNAs and conventional antisense-added type ribozymes (Figures 10 and 14), which were targeted to the *BCR-ABL* junction, failed to demonstrate any specificity in mammalian cells. Therefore, in this kind of investigation, it is very important to confirm that cell death does indeed originate from specific suppression by the antisense molecule. This point is at least as important as deductions of the efficacy of inhibition. Since the specificity of our maxizyme was considerable and since the lengths and sequences of the sensor arms and common stem II are variables that can very easily be adjusted, maxizymes in general should be considered to be a novel class of potentially powerful gene-inactivating agents that should be able to cleave other chimeric mRNAS as well.

The cleaving activity of the maxizyme, in particular in cells, should involve a trimolecular interaction (between the two tRNA^{Val}-driven monomer units of the maxizyme and the target substrate). In contrast, the activity of conventional ribozymes involves a bimolecular interaction (between one tRNA^{Val}-driven ribozyme and its target). In principle, bimolecular interactions are more rapid than trimolecular interactions. This difference may seem to indicate that conventional ribozymes might be more effective in cells than a maxizyme. However, in our experiments, we found that the tRNA^{Val}-driven dimer was always more active than the corresponding tRNA^{Val}-driven ribozymne when we tested several target sequences in cultured cells (the same target site was used for each set of ribozyme and maxizyme). This conclusion is further strengthened by the results of the present analysis. The maxizyme cleaved the junction in L6 *BCR-ABL* mRNA more effectively than the ribozyme, not only reporter constructs (Figure 14) but also when the target was endogenous molecule (Figures 18-21). Therefore, as long as our tRNA^{Val}-expression system is used, despite the involvement of the dimerization process, the intracellular activity of the maxizyme is significantly higher than that of conventional hammerhead ribozymes.

Klug's group demonstrated, in an accurate and elegant experiment, that a carefully designed DNA-binding peptide, which consisted of three zinc-finger motifs, bound specifically to a unique nine-base-pair region of a *BCR-ABL* fusion oncogene in preference to the parent genomic sequences (Choo et al., 1994). Moreover, murine cells that had been rendered independent of growth factors (IL-3) by the action of the oncogene become dependent on IL-3 again upon transient transfection with a vector expressing the DNA-binding peptide. Note that the p210^{*BCR-ABL*} protein does not trigger the endogenous expression of IL-3 or of other growth factors that are capable of stimulating proliferation of BaF3 cells in an autocrine manner. Rather, p210^{*BCR-ABL*} provides the stimulus for proliferation of BaF3 cells that is normally provided through the IL-3 signal transduction pathway (Daley and Baltimore, 1988). Klug's group further demonstrated that levels of *BCR-ABL* mRNA in the transiently transfected cells fell by 15-18% within 24 hours as compared to those in untransfected cells. We found that a similar reduction in the level of *BCR-ABL* mRNA (35% reduction within 24 hours in the case of the maxizyme; Figure 20) restored dependence on IL-3 (Figure 18).

The mechanism by which a deregulated *BCR-ABL* tyrosine kinase delays apoptotic cell death remains poorly understood. Transduction of the apoptotic signal and execution of apoptosis require the coordinated actions of several caspases. The ten human caspases identified to date can be classified into four subfamilies on the basis of structure and the extent of homology to the human prototype interleukin-1β-converting enzyme (ICE) and the nematode prototype CED-3 (Alnemri et al., 1996). All these caspases, which should cause apoptosis when they are overexpressed in cells, are initially synthesized as single-chain inactive proenzymes that require cleavage distal to aspartate residues for generation of the active protease. Recent evidence indicates that activation of caspases in apoptosis occurs via a proteolytic cascade. For example, caspase-4 activates procaspase-1 which, in turn, cleaves procaspase-3 to yield active caspase-3 that recognizes the Asp-Glu-Val-Asp (DEVD) motif and cleaves poly(ADP-ribose) polymerase (Enari et al., 1996). The findings that caspase-1-null mice did not show any phenotype in programmed cell death (Li et al., 1995), while mice lacking caspase-3 showed hyperplasia and the disorganized development of cells in the brain (Kuida et al., 1996), suggests that caspase-1 might be redundant in all types of cells, while caspase-3 appears to play a major role in apoptosis in some parts of the brain (Nagata, 1997). The very recent finding that, in the *BCR-ABL*-mediated inhibition of apoptosis, the apoptotic pathway is interrupted upstream of activation of procaspase-3 in *BCR-ABL*⁺ cell lines (Dubrez et al., 1998) was confirmed in the present study. Depletion of p210^{*BCR-ABL*} as a result of expression of the maxizyme clearly enhanced the processing of inactive procaspase-3 to yield active caspase-3 (Figure 21). The selective cleavage of L6 *BCR-ABL* mRNA by the maxizyme and the eventual activation of caspase-3, which led to apoptosis in leukemic cells but not in normal cells, demonstrated that our designed novel maxizyme was fully functional in cells.

In conclusion, we demonstrated that (1) for cleavage of L6 *BCR-ABL* mRNA, our novel maxizyme formed a heterodimeric structure with high-level activity in cells, and (2) cleaving activity was successfully controlled allosterically within cells such that only in the presence of the junction of L6 *BCR-ABL* mRNA did the maxizyme form an active catalytic core. The maxizyme was more effective than similarly transcribed standard ribozymes in cells. To the best of our knowledge, our novel maxizyme is superior to other nucleic acid-based drugs reported to date because of its extremely high substrate-specificity. A maxizyme of this type may be useful for the treatment of chronic myelogenous leukemia (CML), particularly those caused by L6 translocations.

### [Example 12] Effect of maxizyme in vivo (animal experiment)

Experimental results of this maxizyme indicated that the maxizyme was also effective *in vivo* (in animal experiment level). Briefly, when the maxizyme was introduced into a mouse with chronic myelocytic leukemia, cancer was greatly suppressed.

### Experimental procedure

To develop cancer in mice, cells from a human patient with b2a2 type CML were used (cells from a patient with CML of L6-translocation-type, which have not been established as a cell line obtained from the Department of Pathology, the Institute of Medical Science, the University of Tokyo). These tumor cells were infected with either maxizyme-inserted viral vector pMX puro/Dimer (pV-MzL/R of Examples 8, 10 and 11) or viral vector pMZ puro with no insert (Kitamura et al., 1995) as a control. For specific infection conditions, 1 x 10⁷ of the CML cells were infected with 10 ml viral vector (titer 1 x 10⁵) (m.o.i.: 0.1) on a stroma cell (a bone marrow stroma cell); a fibroblastic adhesion cell which is increased upon culturing cells taken from bone marrow and which secretes cytokine and stimulates proliferation of hemocytes; in the present example, this cell was obtained from the Department of Pathology, the Institute of Medical Science, the University of Tokyo) in the presence of polybrene 10 microgram/ml. Since a puromycine-resistant gene is incorporated in the viral vector, only cells incorporating the maxizyme can be selected by adding 0.5 mg/ml puromycine to the culture solution 72 hours after the infection (selection time: 72 hours). The patient-derived tumor cells introduced with the maxizyme and those without the maxizyme were injected into respective mice. NOD-SCID mice (Non obesity diabetes-systemic combined immunodeficiency mice, which have lower immunocompetence than a conventional SCID mouse, and into which various human cells can be transplanted; in the present example, these mice were obtained from the Department of Pathology, the Institute of Medical Science, the University of Tokyo) were used. To the caudal veins of these mice, 1 x 10⁶ puromycine-selected tumor cells were injected. These mice can be irradiated with radiation before the injection so as to further decrease the immunocompetence, although this was not the case in the present example.

### Results

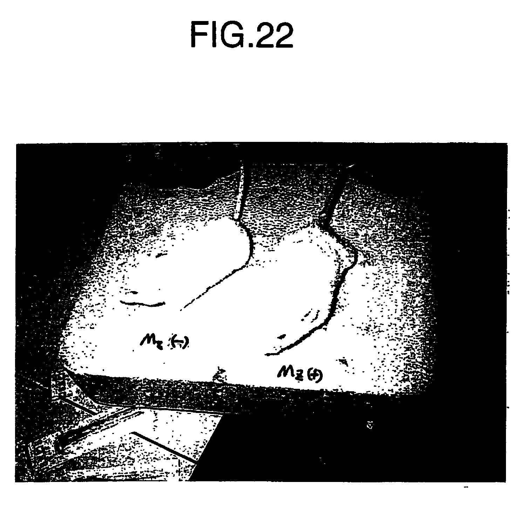

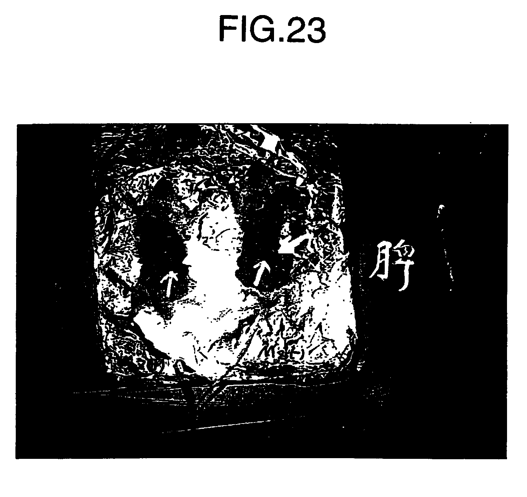

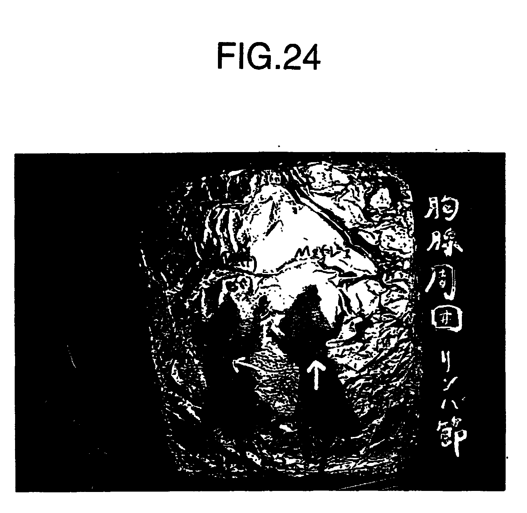

Eleven weeks after the cell injection, the mice were dissected. The results thereof are shown in Figures 22-24. Mz(-) represents the mouse injected with tumor cells without the maxizyme and Mz(+) represents the mouse injected with tumor cells introduced with the maxizyme. Figure 22 shows a picture of mice before dissection (weights of the mice were measured at this point, see Table 1 below). Figures 23 and 24 show pictures of the spleens and lymph nodes around thymi, respectively (as indicated by arrows). As can be appreciated from Figure 22, the Mz(+) mouse grew well and healthy than the Mz(-) mouse. When their weights were actually measured (Table 1), Mz(-) weighed 24 g (one-third the weight of conventional NOD-SCID mouse), while Mz(+) weighed 34 g, differing for as much as 10 g. This indicates that the Mz(-) mouse has developed cancer and reduced weight. Then, the mice were dissected to compare their spleens (Figure 23). While Mz(-) exhibited hypertrophy of the spleen due to obvious cancer development (0.21 g), the mouse introduced with the maxizyme did not exhibit hypertrophy of the spleen (0.08 g). Similarly. Figure 24 shows comparison between the lymph nodes around thymi. The lymph node of the Mz(-) mouse (0.27 g) indicated cancer development as compared to that of the mouse introduced with the maxizyme (0.03 g).

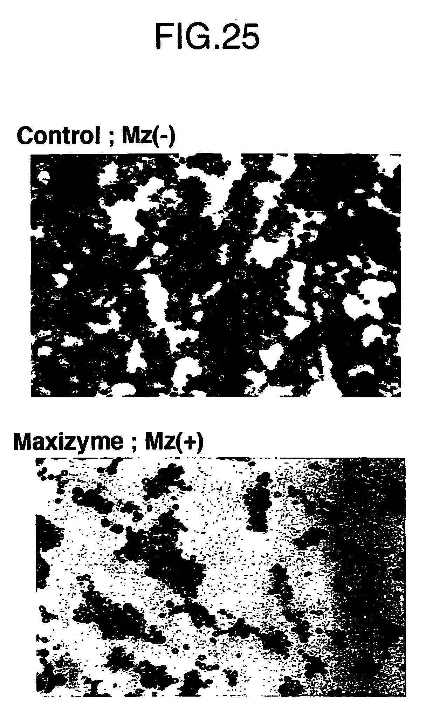

Figure 25 shows pictures of bone marrows of the above-described mice. The bone marrow of the Mz(-) mouse was almost completely occupied by tumor cells, while that of the Mz(+) mouse introduced with the maxizyme was the same as healthy bone marrow (containing diverse cells such as lymphocytes, rather than tumor cells.)

Among the currently employed clinical treatments for leukemia, only bone marrow transplantation has been confirmed of its efficacy at present. However, bone marrow transplantation is not applicable to every patient, and there are insufficient number of bone marrow donors. Thus, a safe therapy which can more generally be employed has been demanded. From the results obtained herein, the maxizyme has very high activity to effectively suppress cancer development of CML and has no toxicity. Since maxizyme can be applied to broader range of patients as compared to conventional treatments, it may greatly contribute to the future gene therapy of chronic myelocytic leukemia.

**Table 1**

| | Weight | Spleen | lymph node around thymus |
|---|---|---|---|
| Control (Mz(-)) | 24 g | 0.21 g | 0.27 g |
| Maxizyme (Mz(+)) | 34 g | 0.08 g | 0.03 g |

### INDUSTRIAL APPLICATION

The maxizyme of the invention can be used to specifically suppress expression of abnormal L6 (b2a2) chimeric mRNA which is produced in chronic myelocytic leukemia triggered by translocations on chromosomes or the like, without giving any influence on normal mRNA. Thus, the maxizyme of the invention can be utilized for gene therapy of chronic myelocytic leukemia and as a suppressor of expression of L6 (b2a2) chimeric mRNA causative of chronic myelocytic leukemia.

### [References]

Zhou, D.-M., Zhang, L.-H., and Taira, K. (1997). Explanation by the double-metal-ion mechanism of catalysis for the differential metal ion-effects in the cleavage rates of 5'-oxy and 5'-thio substrates by a hammerhead ribozyme. Proc. Natl. Acad. Sci. USA 94, 14343-14348.
Zhou, D.-M. and Taira, K. (1998). The hydrolysis of RNA: from theoretical calculations to the hammerhead ribozyme-mediated cleavage of RNA. Chem. Rev. 98. 991-1026. Herbert, E., Shih, W. and Altman, A. (1994). Construction and characterization of lck-and fyn-specific tRNA:ribozyme chimeras. Mol. Immunol., 31, 923-932.
Bedi, A., Zehnbauer, B.A., Barber, J.P., Sharkis, S.J., and Jones, R.J. (1994). Inhibition of apoptosis by BCR-ABL in chronic myelogenous leukemia. Blood 83, 2038-2045.
Bedi, A., Barber, J.P., Bedi, G.C., el-Deiry, W.S., Sidransky, D., Vala, M.S., Akhtar, A.J., Hilton, J., and Jones, R.J. (1995). BCR-ABL-mediated inhibition of apoptosis with delay of G2/M transition after DNA damage : a mechanism of resistance to multiple aniti-cancer agents. Blood 86, 1148-1153.
Bartram, C.R., de Klein, A., Hagemeijer, A., van Agthoven, T., van Kessel. A. G., Bootsma, D., Grosveld, G., Ferguson-Smith, M.A., Davies, T., Stone, M., Heisterkamp, N,, Stephenson, J.R., and Groffen, J. (1983). Translocation of c-abl oncogene correlates with the presence of a Philadelphia chromosome in chronic myelocytic leukemia. Nature 306, 277-280.
Bertrand, E., Castanotto, D., Zhou, C., Carbonnelle, C., Lee, G. P., Chatterjee, S., Grange, T., Pictet, R., Kohn, D., Engelke, D. and Rossi, J.J. (1997) The expression cassette determines the functional activity of ribozymes in mammalian cells by controlling their intracellular localization. RNA 3, 75-88.
Birikh, K.R., Heaton, P.A., and Eckstein, F. (1997). The hammerhead ribozymestructure, function and application. Eur. J. Biochem. 245, 1-16.
Chapman, R.S., Whetton, A.D., and Dive, C. (1994). The suppression of drug-induced apoptosis by activation of v-abl protein tyrosine kinase. Cancer Res. 54, 5131-5137.
Choo, Y., Sanchez-Garcia, I., and Klug, A. (1994). In vivo repression by a site-specific DNA-binding protein designed against an oncogenic sequence. Nature 372, 642-645.
Dahm, S.C, Derrick, W.B. and Uhlenbeck, O.C. (1993) Role of divalent metal ions in the hammerhead RNA cleavage reaction. Biochemistry 30, 9464 -9469.
Daley, G.Q., and Baltimore, D.(1988). Transformation of an interleukin 3-dependent hematopoietic cell line by the chronic myelogenous leukemia-specific P210^{bcr/abl} protein. Proc. Natl. Acad. Sci. USA 85, 9312-9316.
Duberz, L., Eymin, B., Sordet, O., Droin, N., Turhan, A.G. and Solary, E. (1998). BCR-ABL delays apoptosis upstream of procaspase-3 activation. Blood 7, 2415-2422.
Eckstein, F. and Lilley, D.M.J. (eds.)(1996). Catalytic RNA, Nucleic Acids and Molecular Biology, vol. 10. Springer-Verlag, Berlin.
Enari, M., Talanian, R.V., Wong, W.W., and Nagata, S. (1996). Sequential activation of ICE-like and CPP32-like proteases during Fas-mediated apoptosis. Nature 380, 723-726.
Fu, D.J., Benseler, F. and McLaughlin, L.W. (1994). Hammerhead ribozymes containing non-nucleoside linkers are active RNA catalysts. J. Am. Chem. Soc. 116, 4591-4598.
Geiduschek, E.P. and Tocchini-Valentini, G.P. (1988). Transcription by RNA polymerase III. Annu. Rev. Biochem. 57, 873-914.
Groffen, J., Stephenson, J.R., Heisterkamp, N., de Klein, A., Bartram, C.R., and Grosveld, G. (1984). Philadelphia chromosomal break-points are clustered within a limited region, bcr, on chromosome 22. Cell 36, 93-99.
Haseloff, J., and Gerlach, W.L. (1988). Simple RNA enzymes with new and highly specific endonuclease activities. Nature 334, 585-591.
Heisterkamp, N., Stephenson J.R., Groffen, J., Hansen, P.F., de Klein, A., Bartram, C.R., and Grosveld, G. (1983). Localization of the c-abl oncogene adjacent to a translocation break point in chronic myelocytic leukemia. Nature 306, 239-242.
Hertel, K.J., Herschlag, D., and Uhlenbeck. (1996). Specificity of hammerhead ribozyme cleavage. EMBO J. 15, 3751-3757.
Huang. Y. and Carmichael, G.G.(1996). Role of polyadenylation in nucleo-cytoplasmic transport of mRNA. Mol. Cell. Biol. 16, 1534-1542.
James, H., Mills, K., and Gibson, I. (1996). Investigating and improving the specificity of ribozymes directed against the bcr-abl trans-location. Leukemia 10, 1054-1064.
Kawasaki, H., Ohkawa, J., Tanishige, N., Yoshinari, K., Murata, T., Yokoyama, K.K. and Taira, K. (1996). Selection of the best target site for ribozyme-mediated cleavage within a fusion gene for adenovirus ElA-associated 300 kDa protein (p300) and luciferase. Nucleic Acids Res. 24, 3010-3016.
Kawasaki, H., Eckner, R., Yao, T-P., Taira, K., Chiu, R., Livingston, D.M. and Yokoyama, K.K. (1998). Distinct roles of the co-activators p300 and CBP in retinoic-acid-induced F9-cell differentiation. Nature 393, 284-289.
Kazakov, S. and Altman, S. (1992). A trinucleotide can promote metal ion-dependent specific cleavage of RNA. Proc. Natl. Acad. Sci. USA 89, 7939-7943.
Kearney, P., Wright, L.A., Milliken, S., and Biggs, J.C. (1995). Improved specificity of ribozyme-mediated cleavage of bcr-abl mRNA. Exp. Hematol. 23, 986-989.
Kitamura, T., Onishi, M., Kinoshita, S., Shibuya, A., Miyajima, A. and Nolan, G.P. (1995) Efficient screening of retroviral cDNA expression libraries. Proc. Natl. Acad. Sci. USA 92, 9146-9150.
Konopka, J.B., Watanabe, S.M., and Witte, O.N. (1984). An alteration of the human c-abl protein in K562 leukemia cells unmasks associated tyrosine kinase activity. Cell 37, 1035-1042.
Koseki, S., Ohkawa, J., Yamamoto, R., Takebe, Y. and Taira, K. (1998). A simple assay system for examination of the inhibitory potential in viva of decoy RNAs, ribozymes and other drugs by measuring the Tat-mediated transcription of a fusion gene composed of the long terminal repeat of HIV-1 and a gene for luciferase. J. Control. Release 53, 159-173.
Kronenwett, R., Haas, R. and Sczakiel, G. (1996). Kinetic selectivity of complementary nucleic acids: bcr-abl-directed antisense RNA and ribozymes. J. Mol Biol. 259, 632-644.
Kuida, K., Zheng, T.S., Na, S. -Q., Kuan, C. -Y., Yang, F., Karasuyama, H., Rakic, P., and Flavell, R.A. (1996). Decreased apoptosis in the brain and premature lethality in CPP32-deficient mice. Nature 384, 368-372.
Kuwabara, T., Amontov,S., Warashina, M., Ohkawa, J. and Taira, K. (1996). Characterization of several kinds of dimer minizyme:simultaneous cleavage at two sites in HIV-1 tat mRNA by dimer minizymes. Nucleic Acids Res. 24, 2302-2310.
Kuwabara, T., Warashina, M. Tanabe, T., Tani, K., Asano, S., and Taira, K. (1997). Comparison of the specificities and catalytic activi-ties of hammerhead ribozymes and DNA enzymes with respect to the cleavage of BCR-ABL chimeric L6(b2a2) mRNA. Nucleic Acids Res. 25, 3074-3082.
Laneuville, P., Timm, M., and Hudson, A.T. (1994). Bcr/abl expression in 32D c13(G) cells inhibits apoptosis induced by protein tyrosine kinase inhibitors. Cancer Res. 54, 1360-1368.
Lange, W., Cantin, E. M., Finke, J., and Dolken, G. (1993). In vitro and in vivo effects of synthetic ribozymes targeted against BCR/ABL mRNA. Leukemia 7, 1786-1794.
Lange, W., Daskalakis, M., Finke, J., and Dolken, G. (1994). Comparison of different ribozymes for efficient and specific cleavage of BCR/ABL related mRNAs. FEBS Lett. 338. 175-178.
Leopold, L.H., Shore, S.K,, Newkirk, T.A., Reddy, R.M.V., and Reddy, P. (1995). Multi-unit ribozyme-mediated cleavage of bcr-abl mRNA in myeloid leukemias. Blood 85, 2162-2170.
Li, P., Allen, H., Banerjee, S., Franklin, S., Herzog, L., Johnson, C., McDowell, J., Paskind, M., Rodman, L., Salfeld, J., et al. (1995). Mice deficient in IL-1b-converting enzyme are defective in production of mature IL-1b and resistant to endotoxic shock. Cell 80, 401-411.
Long, D.M. and Uhlenbeck, O.C. (1994). Kinetic characterization of intramolecular and intermolecular hammerhead RNAs with stem II deletions. Proc. Natl. Acad. Sci. USA 91, 6977-6981.
Maekawa, T., Kimura, S., Hirakawa, K., Murakami, A., Zon, G., and Abe, T. (1995). Sequence specificity on the growth suppression and induction of apoptosis of chronic myeloid leukemia cells by BCR-ABL anti-sense oligonucleoside phosphorothioates. Int. J. Cancer 62, 63 -69.
Mahon, F.X., Ripoche, J., Pigeonnier, V., Jazwiec, B., Pigneux, A., Moreau, J.F., and Reiffers, J. (1995). Inhibition of chronic myelogenous leukemia cells harboring a BCR-ABL B3A2 junction by antisense oligonucleotides targeted at the B2A2 junction. Exp. Hematol. 23, 1606-1611.
McCall, M. J., Hendry, P. and Jennings, P.A. (1992). Minimal sequence requirements for ribozyme activity. Proc. Natl. Acad. Sci. USA 89, 5710-5714.
McGahon, A., Bissonnette, R., Schmitt, M., Cotter, K.M., Green, D. R., and Cotter, T.G. (1994). BCR-ABL maintains resistance of chronic myelogenous leukemia cells to apoptotic cell death. Blood 83, 1179-1187.
McGahon, A,J., Nishioka, W.K., Martin, S.J., Mahboubi, A., Cotter, T.G., and Green, D.R. (1995). Regulation of the Fas apoptotic cell death pathway by abl. J. Biol. Chem. 270, 22625-22631.
Muller, A.J., Young, J.C., Pendergast, A.M., Pondel, M., Landau, N.R., Littman, D.R., and Witte, O.N. (1991). BCR first exon sequences specifically activate the BCR/ABL tyrosine kinase oncogene of Philadelphia chromosome-positive human leukemias. Mol. Cell. Biol. 11, 1785-1792.
Nagata, S. (1997). Apoptosis by death factor. Cell 88, 355-365.
Nowell, P.C., and Hungerford, D.A. (1960). A minute chromosome in human chronic granulocytic leukemia. Science 132, 1497-1499.
O'Brien, S.G., Kirkland, M.A., Melo, J.V., Rai, M.H., Davidson, R.J., McConald, V., and Goldman, J.M. (1994). Antisense BCR-ABL oligomers cause non-specific inhibition of chronic myeloid leukemia cell lines. Leukemia 8, 2156-2162.
Pachuk, C.J., Yoon, K., Moelling, K., and Coney, L.R. (1994). Selective cleavage of bcr-abl chimeric RNAs by a ribozyme targeted to non-conitiguous sequence. Nucleic Acids Res. 22, 301-307.
Pendergast, A.M., Gishizky, M.L., Havlik, M.H., and Witte, O.N. (1993). SH1 domain autophosphorylation of p210 BCR/ABL is required for transformation but not growth factor independence. Mol. Cell. Biol. 13, 1728-1736.
Perriman, R. and de Feyter, R. (1997). tRNA-delivery systems for ribozymes. In Turner, P.C. (ed.), Mrthods in Molecular Biology, Ribozyme Protocols. Humana Press, Totowa, NJ, pp.393-402.
Piccirili, J.A., Vyle, J.S., Caruthers, M.H. and Cech, T.R. (1993). Metal ion catalysis in the Tetrahymena ribozyme reaction. Nature 361, 85-88.
Porta, H., and Lizardi, P.M. (1995). An allosteric hammerhead ribozyme. Biotechnology 13, 161-164.
Reuther, J.Y., Reuther, G.W, Cortez, D., Pendergast, A.M., and Baldwin, Jr., A.S. (1998). A requirement for NF-kB activation in Bcr-Abl-mediated transformation. Genes & Dev. 12, 968-981.
Rowley, J.D. (1973). Letter: A new consistent chromosomal abnormality in chronic myelogenous leukemia identified by quinacrine fluorescence and Giemsa staining. Nature 243, 290-293.
Sawata, S., Komiyama, M., and Taira, K. (1995). Kinetic evidence based on solvent isotope effects for the nonexistence of a proton-transfer process in reactions catalyzed by a hammerhead ribozyme: implication to the double-metal-ion mechanism of catalysis. J.Am. Chem. Soc. 117, 2357-2358.
Shore, S.K., Nabissa, P.M., and Reddy, E.P. (1993). Ribozyme-mediated cleavage of the BCR-ABL oncogene transcript: in vitro cleavage of RNA and in vivo loss of P210 protein-kinase activity, Oncogene 8, 3183-3188.
Shtivelman, E., Lifshitz, B., Gale, R.P., and Canaani, E. (1985). Fused transcript of abl and bcr genes in chronic myelogenous leukemia. Nature 315, 550-553.
Shtivelman, E., Lifschitz, B., Gale, R. P., Roe, B.A., and Canaani, J. (1986). Alternative splicing of RNAs transcribed from the human abl gene and from the bcr-abl fused gene. Cell 47, 277-284.
Smetsers, T.F., van de Locht, L.T., Pennins, A.H., Wessels, H.M., de Witte, T.M., and Mensink, E.J. (1995). Phosporothioate BCR-ABL antisense oligonucleotides induce cell death, but fail to reduce cellular bcr-abl protein levels. Leukemia 9, 118-130.
Smetsers, T.F., Linders, E.H., van de Locht, L.T., de Witte, T.M., and Mensink, E.J. (1997) An antisense Bcr-Abl phosphodiester-tailed methylphosphonate oligonucleotide reduces the growth of chronic myeloid leukemia patient cells by a non-antisense mechanism. Br. J.Hematol. 96, 377-381.
Snyder, D.S., Wu, Y., Wang, J.L., Rossi, J.J., Swiderski, P., Kaplan, B.E., and Forman, S.J. (1993). Ribozyme-mediated inhibition of bcr-abl gene expression in a Philadelphia chromosome-positive cell line. Blood 82, 600-605.
Spooncer, E., Fairbairn, L., Cowling. G.J., Dexter, T.M., Whetton, A.D., and Owen-Lynch, P.J. (1994). Biological consequences of p160 v-abl protein tyrosine kinase activity in primitive, multipotent hematopoietic cell line. Leukemia 8, 620-626.
Steitz, T.A. and Steitz, J.A. (1993). A general two-metal-ion mechanism for catalytic RNA, Proc. Natl. Acad. Sci. USA 90, 6498-6502.
Sullenger, B.A. and Cech, T.R. (1993). Tethering ribozymes to a retroviral packaging signal for destruction of viral RNA. Science 262, 1566-1569.
Symons, R.H. (1989). Self-cleavage of RNA in the replication of small pathogens of plants and animals. Trends Biochem. Sci. 14, 445-450.
Tang, J., and Breaker, R.R. (1997a). Rational design of allosteric ribozymes. Chem. Biol. 4, 453-459.
Tang, J., and Breaker, R.R. (1997b). Examination of the catalytic fitness of the hammerhead ribozyme by in vitro selection. RNA 3, 914-925.
Terns, M., Dahlberg, J., Lund, E. (1993). Multiple cis-acting signals for export of pre-UI snRNA from the nucleus. Genes & Dev. 7, 1898- 1908.
Tuschl, T., and Eckstein, F. (1993). Hammerhead ribozymes: importance of stem-loop II activity. Proc. Natl. Acad. Sci. USA 90, 6991-6994.
Uhlenbeck, O.C. (1987). A small catalytic oligonucleotide. Nature 328, 596-600.
Yu, M., Leavitt, M.C., Maruyama, M., Yamada, O., Young, D., Ho. A.D., and Wong-Staal, F. (1995). Intracellular immunization of human fetal cord blood stem/progenitor cells with a ribozyme against human immunodeficiency virus type 1. Proc. Natl. Acad. Sci. USA 92, 699-703.
Vaerman, J.L., Lammineur, C., Moureau, P., Lewalle, P., Deldime, F., Blumanfeld, M., and Martiat, P. (1995) BCR-ABL antisense oligodeoxyribonucleotides suppress the growth of leukemic and normal hematopoietic cells by a sequence-specific but nonantisense mechanism. Blood 86, 3891-3896.
Vaerman, J.L., Moureau, P., Deldime, F., Lewalle, P., Lammineur, C., Morschhauser, F., and Martiat, P. (1997). Antisense oligodeoxyribonucleotides suppress hematologic cell growth through stepwise release of deoxyribonucleotides Blood 90, 331-339.
Wright, L., Wilson, S.B., Milliken, S., Biggs,J., and Kearney, P. (1993). Ribozyme-mediated cleavage of the bcr/abl transcript expressed in chronic myeloid leukemia. Exp. Hematol. 21, 1714-1718.
Zhou, D.-M., Zhang, L.-H., and Taira, K. (1997). Explanation by the double-metal-ion mechanism of catalysis for the differential metal ion-effects in the cleavage rates of 5'-oxy and 5'-thio substrates by a hammerhead ribozyme. Proc. Natl. Acad. Sci. USA 94, 14343-14348.
Zhou, D.-M. and Taira, K. (1998). The hydrolysis of RNA: from theoretical calculations to the hammerhead ribozyme-mediated cleavage of RNA. Chem. Rev. 98, 991-1026.

All publications, patents and patent applications referred to herein will be incorporated herein as references.

## Claims

1. A nucleic acid enzyme exhibiting allosteric RNA-cleaving activity on a target RNA.

2. A nucleic acid enzyme according to claim 1, comprising a dimeric structure formed by an RNA molecule containing the following nucleotide sequence (10) and an RNA molecule containing the following nucleotide sequence (20),
5'X¹ₗ...X¹ₕ Y¹ₗ...Y¹ᵢ Z¹ₗ...Z¹ⱼ 3' (10)
5'Z²ₗ...Z²ₙ Y²ₗ...Y²ₘ X²ₗ...X²ₖ 3' (20)
(wherein X¹ₗ-X¹ₕ, X²ₗ-X²ₖ, Y¹ₗ-Y¹ₗ, Y²ₗ-Y²ₘ, Z¹ₗ-Z¹ⱼ and Z²ₗ-Z²ₙ are independently any one of A, U, T, C and G;
h and k are integers of 1 or higher;
i and m are integers of 1 or higher;
j is an integer of 1 or higher;
n is an integer of 1 or higher;
X¹ₗ...X¹ₕ and X²ₗ...X²ₖ are nucleotide sequences complementary to a specific sequence in the target RNA;
Y¹ₗ...Y¹ᵢ and Y²ₗ...Y²ₘ are nucleotide sequences forming stems; and
Z¹ₗ...Z¹ⱼ and Z²ₗ...Z²ₙ are nucleotide sequences containing a region complementary to a sequence near a cleavage site of the target RNA and a region capable of forming a cavity for capturing Mg²⁺ ion only in the presence of the target RNA).

3. A nucleic acid enzyme according to claim 1 or 2, wherein the target RNA is a chimeric mRNA causative of a disease.

4. A nucleic acid enzyme according to claim 3, wherein the chimeric mRNA is L6 (b2a2) chimeric mRNA causative of chronic myelocytic leukemia.

5. A nucleic acid enzyme according to claim 4, comprising a dimeric structure formed by an RNA molecule containing the following nucleotide sequence (1) and an RNA molecule containing the following nucleotide sequence (2),
5'GAAGGGCUUC UUUCAUCGAA ACCCUGAGG 3' (1) (SEQ ID NO:1)
5'CACUCACUGA UGAGAGUUAU UGAUGGUCAG 3' (2) (SEQ ID NO:2)
(wherein nucleotides 21-29 of the nucleotide sequence (1) and nucleotides 17-31 of the nucleotide sequence (2) may be modified to conform complementation with the sequence near the cleavage site of the target RNA).

6. A nucleic acid enzyme according to claim 5, wherein a linker sequence and a tRNA^{Val} promoter sequence are added upstream of each of the nucleotide sequences (1) and (2).

7. A nucleic acid enzyme according to claim 6, wherein the linker sequence added upstream of the nucleotide sequence (1) contains the following nucleotide sequence (3), and the linker sequence added upstream of the nucleotide sequence (2) contains the following nucleotide sequence (4),
5'AAA 3' (3)
5'UUU 3' (4).

8. A nucleic acid enzyme according to claim 6, wherein the tRNA^{Val} promoter sequence added upstream of each of the nucleotide sequences (1) and (2) contains the following nucleotide sequence (5),
5'ACCGUUGGUU UCCGUAGUGU AGUGGUUAUC ACGUUCGCCU AACACGCGAA AGGUCCCCGG UUCGAAACCG GGCACUACAA AAACCAAC 3' (5) (SEQ ID NO:3).

9. A nucleic acid enzyme according to claim 6, wherein an additional sequence and a terminator sequence are added downstream of each of the nucleotide sequences (1) and (2).

10. A nucleic acid enzyme according to claim 9, wherein the additional sequence added downstream of the nucleotide sequence
(1) contains the following nucleotide sequence (6), the additional sequence added downstream of the nucleotide sequence
(2) contains the following nucleotide sequence (7), and the terminator sequence added downstream of each of the nucleotide sequences (1) and (2) contains the following nucleotide sequence (8),
5'AAA 3' (6)
5'AACCGUA 3' (7)
5'UUUUU 3' (8).

11. A nucleic acid enzyme according to claim 1 or 2, wherein the target RNA is an abnormal mRNA causative of a disease.

12. An expression vector comprising a DNA coding for the nucleic acid enzyme of any one of claims 1-11.

13. A method for producing the nucleic acid enzyme of claim 1, wherein an expression vector DNA containing DNA encoding the nucleic acid enzyme of claim 1 is used as a template in the transcription to RNA.

14. A pharmaceutical composition comprising the nucleic acid enzyme of any one of claims 1-11 or the expression vector of claim 12 as an effective component.

15. A pharmaceutical composition according to claim 14, for preventing and/or treating a disease caused by the target RNA.

16. A pharmaceutical composition according to claim 15, wherein the nucleic acid enzyme of any one of claims 3-10 is expressed *in vivo* to suppress or inhibit expression of a chimeric mRNA causative of a disease.

17. A pharmaceutical composition according to claim 16, for preventing and/or treating a disease caused by Philadelphia chromosome abnormality.

18. A pharmaceutical composition according to claim 17, wherein the disease caused by Philadelphia chromosome abnormality is chronic myelocytic leukemia.

19. A pharmaceutical composition according to claim 15, wherein the nucleic acid enzyme of claim 11 is expressed to suppress or inhibit expression of an abnormal mRNA causative of a disease.

20. A method for specifically cleaving the target RNA by using the nucleic acid enzyme of claim 1.

21. A method according to claim 20, wherein the target RNA is a chimeric mRNA causative of a disease.

22. A method according to claim 21, wherein the disease is caused by Philadelphia chromosome abnormality.

23. A method according to claim 22, wherein the disease caused by Philadelphia chromosome abnormality is chronic myelocytic leukemia.

24. A method according to claim 20, wherein the target RNA is an abnormal mRNA causative of a disease.
